# EUROPEAN PATENT APPLICATION

(11) **EP 3 757 127 A1**
(43) Date of publication of application: **30.12.2020**
(21) Application number: 19183345.8
(22) Date of filing: 28.06.2019
(51) Int. Cl.: C07K 16/28, A61P 1/00, A61P 25/28

(54) **DEIMMUNIZED ANTIBODIES BINDING TO ALPHA-4 INTEGRIN AND USES THEREOF**

(71) Applicant: Institute for Research in Biomedicine, 6500 Bellinzona (CH)
(72) Inventor: Piccoli, Luca, 6500 Bellinzona (CH); Cassotta, Antonino, 6500 Bellinzona (CH); Lanzavecchia, Antonio, 6948 Porza (CH); Mikol, Vincent, 75008 Paris (FR)
(74) Representative: Graf von Stosch Patentanwaltsgesellschaft mbH

(57) **Abstract**

The present invention provides deimmunized antibodies binding to α4 integrin. In particular, modified versions of natalizumab are provided, in which the immunogenicity is reduced. The invention also provides nucleic acids that encode such antibodies. In addition, the invention provides the use of the antibodies of the invention in prophylaxis and treatment of various diseases including multiple sclerosis and Crohn's disease.

## Description

The present invention relates to the field of α4-integrin binding antibodies, such as natalizumab (NZM).

Natalizumab is a humanized monoclonal IgG4 antibody against the cell adhesion molecule α 4-integrin. In the U.S., natalizumab (NZM) is approved for the treatment of multiple sclerosis and Crohn's disease (Hao, L., Fang-Hong, S., Shi-Ying, H., Shun-Guo, Z. & Min-Ling, C. (2018) A Review on Clinical Pharmacokinetics, Pharmacodynamics, and pharmacogenomics of natalizumab: A Humanized Anti-alpha4 Integrin Monoclonal Antibody. Curr Drug Metab 19(14):1213-1223; Chataway, J. & Miller, D.H. (2013) Natalizumab therapy for multiple sclerosis. Neurotherapeutics 10, 19-28). It is believed that the effects of natalizumab are mediated by reducing the ability of inflammatory immune cells to attach to and pass through the cell layers lining the intestines and blood-brain barrier. Natalizumab has proven effective in treating the symptoms of multiple sclerosis and Crohn's disease, preventing relapse, vision loss, cognitive decline and significantly improving quality of life in people with multiple sclerosis, as well as increasing rates of remission and preventing relapse in multiple sclerosis.

Natalizumab (NZM) is described, for example, in the KEGG DRUG Database (entry: D06886; see also, e.g., CAS Registry Number 189261-10-7, FDA UNII 3JB47N2Q2P). The heavy chain amino acid sequence of NZM is set forth in SEQ ID NO: 25 and the light chain amino acid sequence of NZM is set forth in SEQ ID NO: 31. Accordingly, NZM comprises heavy chain CDR1, CDR2, CDR3 and light chain CDR1, CDR2 and CDR3 sequences as set forth in SEQ ID NOs 1, 2, 3, 4, 11 and 6, respectively. The heavy chain variable region and light chain variable region sequences of NZM are set forth in SEQ ID NOs 15 and 21, respectively.

However, the therapeutic use of monoclonal antibodies and other biopharmaceutical products can result in an immune response to the drug that, in some cases, affects its efficacy due to the production of neutralizing anti-drug antibodies (ADAs; Rup, B., et al. (2015) Standardizing terms, definitions and concepts for describing and interpreting unwanted immunogenicity of biopharmaceuticals: recommendations of the Innovative Medicines Initiative ABIRISK consortium. Clin Exp Immunol 181, 385-400). In particular, natalizumab (NZM), is not a fully human antibody, but a humanized antibody developed on the basis of a murine antibody, which may contribute to its immunogenicity. Natalizumab (NZM) was shown to induce the production of neutralizing anti-drug antibodies (ADAs) in about 9% of the cases leading to therapy discontinuation (in 6% persistent metabolizing antibodies are induced, in 3% they can be transient; Calabresi, P.A., et al. (2007) The incidence and significance of anti-natalizumab antibodies: results from AFFIRM and SENTINEL. Neurology 69, 1391-1403; Bachelet, D., et al. (2016) Occurrence of Anti-Drug Antibodies against Interferon-Beta and Natalizumab in Multiple Sclerosis: A Collaborative Cohort Analysis. PLoS One 11, e0162752).

In view of the above, it is the object of the present invention to overcome the drawbacks of natalizumab. Accordingly, it is the object of the present invention to provide "deimmunized" variants of natalizumab. In particular, it is the object of the present invention to provide NZM variants, in which the binding affinity to MHC molecules is decreased while binding to α4-integrin is maintained.

This object is achieved by means of the subject-matter set out below and in the appended claims.

Although the present invention is described in detail below, it is to be understood that this invention is not limited to the particular methodologies, protocols and reagents described herein as these may vary. It is also to be understood that the terminology used herein is not intended to limit the scope of the present invention which will be limited only by the appended claims. Unless defined otherwise, all technical and scientific terms used herein have the same meanings as commonly understood by one of ordinary skill in the art.

In the following, the elements of the present invention will be described. These elements may be listed with specific embodiments, however, it should be understood that they can be combined in any manner and in any number to create additional embodiments. The variously described examples and embodiments should not be construed to limit the present invention to only the explicitly described embodiments. This description should be understood to support and encompass embodiments which combine the explicitly described embodiments with any number of the disclosed elements. Furthermore, any permutations and combinations of all described elements in this application should be considered disclosed by the description of the present application unless the context indicates otherwise.

Throughout this specification and the claims which follow, unless the context requires otherwise, the term "comprise", and variations such as "comprises" and "comprising", will be understood to imply the inclusion of a stated member, integer or step but not the exclusion of any other non-stated member, integer or step. The term "consist of" is a particular embodiment of the term "comprise", wherein any other non-stated member, integer or step is excluded. In the context of the present invention, the term "comprise" encompasses the term "consist of". The term "comprising" thus encompasses "including" as well as "consisting" *e.g*., a composition "comprising" X may consist exclusively of X or may include something additional *e.g.*, X + Y.

The terms "a" and "an" and "the" and similar reference used in the context of describing the invention (in particular in the context of the claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. No language in the specification should be construed as indicating any non-claimed element essential to the practice of the invention.

The word "substantially" does not exclude "completely" *e.g*., a composition which is "substantially free" from Y may be completely free from Y. Where necessary, the word "substantially" may be omitted from the definition of the invention.

The term "about" in relation to a numerical value x means x ± 10%, including, for example, x ± 5% or x ± 7%.

The term "disease" as used herein is intended to be generally synonymous, and is used interchangeably with, the terms "disorder" and "condition" (as in medical condition), in that all reflect an abnormal condition of the human or animal body or of one of its parts that impairs normal functioning, is typically manifested by distinguishing signs and symptoms, and causes the human or animal to have a reduced duration or quality of life.

As used herein, reference to "treatment" of a subject or patient is intended to include prevention, prophylaxis, attenuation, amelioration and therapy. The term "prevention" refers in particular to "prophylactic settings" (e.g., administration of a drug before diagnosis or "in advance") and may be used interchangeably with the term "prophylaxis". The terms "subject" or "patient" are used interchangeably herein to mean all mammals including humans. Examples of subjects include humans, cows, dogs, cats, horses, goats, sheep, pigs, and rabbits. In some embodiments, the patient is a human.

Doses are often expressed in relation to the bodyweight. Thus, a dose which is expressed as [g, mg, or other unit]/kg (or g, mg etc.) usually refers to [g, mg, or other unit] "per kg (or g, mg etc.) bodyweight", even if the term "bodyweight" is not explicitly mentioned.

The term "binding" and similar reference means in particular "specifically binding", which does not encompass non-specific sticking.

As used herein, the term "antibody" encompasses various forms of antibodies including, without being limited to, whole antibodies, antibody fragments (such as antigen binding fragments), human antibodies, chimeric antibodies, humanized antibodies, recombinant antibodies and genetically engineered antibodies (variant or mutant antibodies) as long as the characteristic properties according to the invention are retained. In some embodiments, the antibody is a monoclonal antibody. In some embodiments, the antibody does not occur in nature, such as an engineered antibody.

As described above, the term "antibody" generally also includes antibody fragments. Fragments of the antibodies may retain the antigen-binding activity of the antibodies. Such fragments are referred to as "antigen-binding fragments". Antigen-binding fragments include, but are not limited to, single chain antibodies, Fab, Fab', F(ab')2, Fv or scFv. Fragments of the antibodies can be obtained from the antibodies by methods that include digestion with enzymes, such as pepsin or papain, and/or by cleavage of disulfide bonds by chemical reduction. Alternatively, fragments of the antibodies can be obtained by recombinant means, for example by cloning and expressing a part (fragment) of the sequences of the heavy and/or light chain. The invention also encompasses single-chain Fv fragments (scFv) derived from the heavy and light chains of an antibody of the invention. For example, the invention includes a scFv comprising the CDRs from an antibody of the invention. Also included are heavy or light chain monomers and dimers, single domain heavy chain antibodies, single domain light chain antibodies, as well as single chain antibodies, *e.g*., single chain Fv in which the heavy and light chain variable domains are joined by a peptide linker. Antibody fragments of the invention may be contained in a variety of structures known to the person skilled in the art. In addition, the sequences of the invention may be a component of multispecific molecules in which the sequences of the invention target the epitopes of the invention and other regions of the molecule bind to other targets. Although the specification, including the claims, may, in some places, refer explicitly to antigen binding fragment(s), antibody fragment(s), variant(s) and/or derivative(s) of antibodies, it is understood that the term "antibody" includes all categories of antibodies, namely, antigen binding fragment(s), antibody fragment(s), variant(s) and derivative(s) of antibodies.

Human and humanized antibodies are well-known in the state of the art (van Dijk, M. A., and van de Winkel, J. G., Curr. Opin. Chem. Biol. 5 (2001) 368-374; Harrison, Charlotte (2014) The full repertoire of humanized antibodies. Nature Reviews Drug Discovery 13: 336). Human antibodies can also be produced in transgenic animals (e.g., mice) that are capable, upon immunization, of producing a full repertoire or a selection of human antibodies in the absence of endogenous immunoglobulin production. Transfer of the human germ-line immunoglobulin gene array in such germ-line mutant mice will result in the production of human antibodies upon antigen challenge (see, e.g., Jakobovits, A., et al., Proc. Natl. Acad. Sci. USA 90 (1993) 2551-2555; Jakobovits, A., et al., Nature 362 (1993) 255-258; Bruggemann, M., et al., Year Immunol. 7 (1993) 3340). Human antibodies can also be produced in phage display libraries (Hoogenboom, H. R., and Winter, G., J. Mol. Biol. 227 (1992) 381-388; Marks, J. D., et al., J. Mol. Biol. 222 (1991) 581-597). The techniques of Cole et al. and Boerner et al. are also available for the preparation of human monoclonal antibodies (Cole et al., Monoclonal Antibodies and Cancer Therapy, Alan R. Liss, p. 77 (1985); and Boerner, P., et al., J. Immunol. 147 (1991) 86-95). In some embodiments, human monoclonal antibodies are prepared by using improved EBV-B cell immortalization as described in Traggiai E, Becker S, Subbarao K, Kolesnikova L, Uematsu Y, Gismondo MR, Murphy BR, Rappuoli R, Lanzavecchia A. (2004): An efficient method to make human monoclonal antibodies from memory B cells: potent neutralization of SARS coronavirus. Nat Med. 10(8):871-5. Methods for producing humanized antibodies are well-known in the art and described, for example, in Safdari Y, Farajnia S, Asgharzadeh M, Khalili M. (2013) Antibody humanization methods - a review and update. Biotechnol Genet Eng Rev.29:1 75-86).

In general, antibodies, or an antigen-binding fragments thereof, typically comprises (at least) three complementarity determining regions (CDRs) on a heavy chain and (at least) three CDRs on a light chain. In general, complementarity determining regions (CDRs) are the hypervariable regions present in heavy chain variable domains and light chain variable domains. Typically, the CDRs of a heavy chain and the connected light chain of an antibody together form the antigen receptor. Usually, the three CDRs (CDR1, CDR2, and CDR3) are arranged non-consecutively in the variable domain. Since antigen receptors are typically composed of two variable domains (on two different polypeptide chains, i.e. heavy and light chain: heavy chain variable region (VH) and light chain variable region (VL)), there are typically six CDRs for each antigen receptor (heavy chain: HCDR1, HCDR2, and HCDR3; light chain: LCDR1, LCDR2, and LCDR3). A classical single antibody molecule has usually two antigen receptors and therefore contains twelve CDRs. The CDRs on the heavy and/or light chain may be separated by framework regions, whereby a framework region (FR) is a region in the variable domain which is less "variable" than the CDR. For example, a variable region (namely, the heavy chain variable region (VH) and/or the light chain variable region (VL)) may be composed of four framework regions, separated by three CDR's. The position of the CDRs can be defined according to the IMGT numbering system (IMGT: http://www.imgt.org/; cf. Lefranc, M.-P. et al. (2009) Nucleic Acids Res. 37, D1006-D1012). Antibodies of the invention can be of any isotype (*e.g*., IgA, IgG, IgM i.e. an α, γ or µ heavy chain). For example, the antibody is of the IgG type. Within the IgG isotype, antibodies may be IgG1, IgG2, IgG3 or IgG4 subclass, for example IgG4. Antibodies of the invention may have a κ or a λ light chain. In some embodiments, the antibody is of IgG4 type and/or has a κ light chain.

Antibodies according to the present invention may be provided in purified form. Typically, the antibody will be present in a composition that is substantially free of other polypeptides *e.g*., where less than 90% (by weight), usually less than 60% and more usually less than 50% of the composition is made up of other polypeptides. Antibodies can be used alone, or in combination, as prophylactic or therapeutic agents upon appropriate formulation, in association with active vaccination, as a diagnostic tool, or as a production tool as described herein.

As used herein, the term "mutation" relates to a change in the nucleic acid sequence and/or in the amino acid sequence in comparison to a reference sequence. A mutation, e.g. in comparison to a reference sequence, may be, for example, a (naturally occurring) somatic mutation, a spontaneous mutation, an induced mutation, e.g. induced by enzymes, chemicals or radiation, or a mutation obtained by site-directed mutagenesis (molecular biology methods for making specific and intentional changes in the nucleic acid sequence and/or in the amino acid sequence). Thus, the terms "mutation" or "mutating" shall be understood to also include physically making a mutation, e.g. in a nucleic acid sequence or in an amino acid sequence. A mutation includes substitution, deletion and insertion of one or more nucleotides or amino acids as well as inversion of several successive nucleotides or amino acids. To achieve a mutation in an amino acid sequence, a mutation may be introduced into the nucleotide sequence encoding said amino acid sequence in order to express a (recombinant) mutated polypeptide. A mutation may be achieved e.g., by altering (for example by site-directed mutagenesis) a codon of a nucleic acid molecule encoding one amino acid to result in a codon encoding a different amino acid, or by synthesizing a sequence variant, e.g., by knowing the nucleotide sequence of a nucleic acid molecule encoding a polypeptide and by designing the synthesis of a nucleic acid molecule comprising a nucleotide sequence encoding a variant of the polypeptide without the need for mutating one or more nucleotides of a nucleic acid molecule. Sequences containing mutations (in comparison to a reference sequence) are also referred to herein as "variant sequences" or "variants".

While it is possible to have non-conservative amino acid substitutions in a "sequence variant", it is preferred in a "sequence variant" that the substitutions are conservative amino acid substitutions, in which the substituted amino acid has similar structural or chemical properties with the corresponding amino acid in the reference sequence. By way of example, conservative amino acid substitutions involve substitution of one aliphatic or hydrophobic amino acid, e.g. alanine, valine, leucine and isoleucine, with another; substitution of one hydoxyl-containing amino acid, e.g. serine and threonine, with another; substitution of one acidic residue, e.g. glutamic acid or aspartic acid, with another; replacement of one amide-containing residue, e.g. asparagine and glutamine, with another; replacement of one aromatic residue, e.g. phenylalanine and tyrosine, with another; replacement of one basic residue, e.g. lysine, arginine and histidine, with another; and replacement of one small amino acid, e.g., alanine, serine, threonine, methionine, and glycine, with another.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include the fusion to the N- or C-terminus of an amino acid sequence to a reporter molecule or an enzyme.

Several documents are cited throughout the text of this specification. Each of the documents cited herein (including all patents, patent applications, scientific publications, manufacturer's specifications, instructions, etc.), whether supra or infra, are hereby incorporated by reference in their entirety. Nothing herein is to be construed as an admission that the invention is not entitled to antedate such disclosure by virtue of prior invention.

It is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

### Deimmunized antibodies and antigen-binding fragments thereof

In a first aspect, the present invention provides deimmunized antibody variants of natalizumab (NZM), and antigen-binding fragments thereof. Accordingly, an antibody of the invention, or an antigen-binding fragment thereof, contains at least one (i.e., one or more, e.g. 2, 3, 4, 5, 6, 7, 8, 9, 10, 11, 12, 13, 14, 15, 16, 17, 18, 19, 20 or more) amino acid mutation in comparison to natalizumab (NZM). Natalizumab (NZM) is described, for example, in the KEGG DRUG Database (entry: D06886; see also, e.g., CAS Registry Number 189261-10-7, FDA UNII 3JB47N2Q2P). The heavy chain amino acid sequence of NZM is set forth in SEQ ID NO: 25 and the light chain amino acid sequence of NZM is set forth in SEQ ID NO: 31. Accordingly, NZM comprises heavy chain CDR1, CDR2, CDR3 and light chain CDR1, CDR2 and CDR3 sequences as set forth in SEQ ID NOs 1, 2, 3, 4, 11 and 6, respectively. The heavy chain variable region and light chain variable region sequences of NZM are set forth in SEQ ID NOs 15 and 21, respectively.

Accordingly, the antibody of the invention, or the antigen-binding fragment thereof, may comprise a heavy chain amino acid sequence comprising at least one (i.e., one or more, e.g. 2, 3, 4, 5, 6, 7, 8, 9, 10, 11, 12, 13, 14, 15, 16, 17, 18, 19, 20 or more) amino acid mutation in comparison to the amino acid sequence of SEQ ID NO: 25 and/or a light chain amino acid sequence comprising at least one (i.e., one or more, e.g. 2, 3, 4, 5, 6, 7, 8, 9, 10, 11, 12, 13, 14, 15, 16, 17, 18, 19, 20 or more) amino acid mutation in comparison to the amino acid sequence of SEQ ID NO: 31. Moreover, the antibody of the invention, or the antigen-binding fragment thereof, may comprise a heavy chain variable region (VH) amino acid sequence comprising at least one (i.e., one or more, e.g. 2, 3, 4, 5, 6, 7, 8, 9, 10, 11, 12, 13, 14, 15, 16, 17, 18, 19, 20 or more) amino acid mutation in comparison to the amino acid sequence of SEQ ID NO: 15 and/or a light chain variable region (VL) amino acid sequence comprising at least one (i.e., one or more, e.g. 2, 3, 4, 5, 6, 7, 8, 9, 10, 11, 12, 13, 14, 15, 16, 17, 18, 19, 20 or more) amino acid mutation in comparison to the amino acid sequence of SEQ ID NO: 21. In some embodiments the antibody of the invention, or the antigen-binding fragment thereof, comprises an HCDR1 amino acid sequence comprising one or more (e.g., 2, 3, 4, 5 or more) amino acid mutation in comparison to the amino acid sequence of SEQ ID NO: 1, while in other embodiments the antibody, or the antigen-binding fragment thereof, comprises an HCDR1 amino acid sequence as set forth in SEQ ID NO: 1. In some embodiments the antibody of the invention, or the antigen-binding fragment thereof, comprises an HCDR2 amino acid sequence comprising one or more (e.g., 2, 3, 4, 5 or more) amino acid mutation in comparison to the amino acid sequence of SEQ ID NO: 2, while in other embodiments the antibody, or the antigen-binding fragment thereof, comprises an HCDR2 amino acid sequence as set forth in SEQ ID NO: 2. In some embodiments the antibody of the invention, or the antigen-binding fragment thereof, comprises an HCDR3 amino acid sequence comprising one or more (e.g., 2, 3, 4, 5 or more) amino acid mutation in comparison to the amino acid sequence of SEQ ID NO: 3, while in other embodiments the antibody, or the antigen-binding fragment thereof, comprises an HCDR3 amino acid sequence as set forth in SEQ ID NO: 3. In some embodiments the antibody of the invention, or the antigen-binding fragment thereof, comprises an LCDR1 amino acid sequence comprising one or more (e.g., 2, 3, 4, 5 or more) amino acid mutation in comparison to the amino acid sequence of SEQ ID NO: 4, while in other embodiments the antibody, or the antigen-binding fragment thereof, comprises an LCDR1 amino acid sequence as set forth in SEQ ID NO: 4. In some embodiments the antibody of the invention, or the antigen-binding fragment thereof, comprises an LCDR2 amino acid sequence comprising one or more (e.g., 2, 3, 4, 5 or more) amino acid mutation in comparison to the amino acid sequence of SEQ ID NO: 11, while in other embodiments the antibody, or the antigen-binding fragment thereof, comprises an LCDR2 amino acid sequence as set forth in SEQ ID NO: 11. In some embodiments the antibody of the invention, or the antigen-binding fragment thereof, comprises an LCDR3 amino acid sequence comprising one or more (e.g., 2, 3, 4, 5 or more) amino acid mutation in comparison to the amino acid sequence of SEQ ID NO: 6, while in other embodiments the antibody, or the antigen-binding fragment thereof, comprises an LCDR3 amino acid sequence as set forth in SEQ ID NO: 6. Different embodiments of HCDR1, HCDR2, HCDR3, LCDR1, LCDR2 and LCDR3 as described above may be combined in the antibody of the invention, or the antigen-binding fragment thereof.

Except for the above described mutations, the amino acid sequence of the antibody, or the antigen-binding fragment thereof, may correspond to that of NZM.

In particular, the antibody of the invention, or the antigen-binding fragment thereof, is a "deimmunized" antibody, i.e. an antibody exhibiting less immunogenicity than natalizumab (NZM). For example, the antibody, or the antigen-binding fragment thereof, elicits less anti-drug antibodies (ADA) as compared to NZM. In some embodiments, the binding affinity of the antibody, or the antigen-binding fragment thereof, to an MHC molecule is decreased in comparison to the binding affinity of NZM to the same MHC molecule (assessed under the same conditions). Otherwise, the antibody, or the antigen-binding fragment thereof, may bind to the same target as NZM.

In some embodiments, the present invention provides an antibody, or an antigen-binding fragment thereof, binding to α4 integrin, wherein the antibody, or the antigen-binding fragment thereof, is a "deimmunized" antibody.

In particular, the present invention provides an antibody, or an antigen-binding fragment thereof, comprising:
- a heavy chain CDR1 (HCDR1) comprising the amino acid sequence GFNIKDTYIH (SEQ ID NO: 1);
- a heavy chain CDR2 (HCDR2) comprising the amino acid sequence RIDPANGYTKYDPK (SEQ ID NO: 2);
- a heavy chain CDR3 (HCDR3) comprising the amino acid sequence EGYYGNYGVYAMDY (SEQ ID NO: 3);
- a light chain CDR1 (LCDR1) comprising the amino acid sequence KTSQDINKYMA (SEQ ID NO: 4);
- a light chain CDR2 (LCDR2) comprising the amino acid sequence YX₁X₂X₃X₄X₅P, wherein
   X₁ may be any amino acid, however, if X₂ is S, X₃ is A, X₄ is L and X₅ is Q, then X₁ is not T;
   X₂ may be any amino acid, however, if X₁ is T, X₃ is A, X₄ is L and X₅ is Q, then X₂ is not S;
   X₃ may be any amino acid, however, if X₁ is T, X₂ is S, X₄ is L and X₅ is Q, then X₃ is not A;
   X₄ may be any amino acid, however, if X₁ is T, X₂ is S, X₃ is A and X₅ is Q, then X₄ is not L; and
   X₅ may be any amino acid, however, if X₁ is T, X₂ is S, X₃ is A and X₄ is L, then X₅ is not Q
   (SEQ ID NO: 5);
- a light chain CDR3 (LCDR3) comprising the amino acid sequence LQYDNLWT (SEQ ID NO: 6).

In other words, the antibody, or an antigen-binding fragment thereof, comprises (i) the heavy chain CDR1, CDR2, and CDR3 amino acid sequences as set forth in SEQ ID NO: 1, SEQ ID NO: 2, and SEQ ID NO: 3, respectively, and the light chain CDR1, CDR2, and CDR3 amino acid sequences as set forth in SEQ ID NO: 4, SEQ ID NO: 5, and SEQ ID NO: 6, respectively. In particular, the antibody, or the antigen-binding fragment thereof, contains at least one mutation at any one of positions X1, X2, X3, X4 or X5 of SEQ ID NO: 5 in comparison to NZM, while the amino acid sequences of HCDR1, HCDR2, HCDR3, LCDR1 and LCDR3 correspond to those of NZM.

The present inventors have surprisingly found that such antibodies of the invention are "deimmunized" in comparison to NZM, while the functionality of NZM, in particular the binding to α4 integrin, is maintained. In particular, the present inventors identified the LCDR2 of NZM as major source for its undesired immunogenicity, while the amino acids of the LCDR2 of NZM involved in MHC binding are not required for binding to α4 integrin.

In some embodiments, X₁ of SEQ ID NO: 5 may be T or S. In some embodiments, X₅ of SEQ ID NO: 5 may be Q, A, T or S. In particular, X₁ of SEQ ID NO: 5 may be T and/or X₅ of SEQ ID NO: 5 may be Q.

Preferably, the antibody, or the antigen-binding fragment thereof, comprises the light chain CDR2 (LCDR2) amino acid sequence YTX₂X₃X₄QP wherein
X₂ may be any amino acid, however, if X₃ is A and X₄ is L, then X₂ is not S;
X₃ may be any amino acid, however, if X₂ is S and X₄ is L, then X₃ is not A; and
X₄ may be any amino acid, however, if X₂ is S and X₃ is A, then X₄ is not L
(SEQ ID NO: 7).

In some embodiments, (i) X₂ in SEQ ID NO: 5 or 7 is selected from G, T, V, K, D and N; and/or (ii) X₄ in SEQ ID NO: 5 or 7 is selected from N, Q, V, I and S. In particular, X₂ in SEQ ID NO: 5 or 7 may be K and/or X₄ in SEQ ID NO: 5 or 7 may be N.

More preferably, the antibody, or the antigen-binding fragment thereof, comprises the light chain CDR2 (LCDR2) amino acid sequence YTKX3NQP wherein
X₃ may be any amino acid
(SEQ ID NO: 8).

In some embodiments, X₃ in SEQ ID NO: 5 or 7 is selected from A, G, S, T, D, N, V and P. For example, X₃ in SEQ ID NO: 5 or 7 may be selected from A, T, G and S. In particular, X₃ in SEQ ID NO: 5 or 7 may be G or S. In some embodiments, the antibody, or the antigen-binding fragment thereof, comprises the light chain CDR2 (LCDR2) amino acid sequence YTKGNQP (SEQ ID NO: 9). In some embodiments, the antibody, or the antigen-binding fragment thereof, comprises the light chain CDR2 (LCDR2) amino acid sequence YTKSNQP (SEQ ID NO: 10).

The sequences of the heavy chains and light chains of exemplary antibodies of the invention, comprising three different CDRs on the heavy chain and three different CDRs on the light chain were determined. The position of the CDR amino acids were defined according to the IMGT numbering system (IMGT: http://www.imgt.org/; cf. Lefranc, M.-P. et al. (2009) Nucleic Acids Res. 37, D1006-D1012). For example, antibody "NZM var1" comprises the amino acid sequences of HCDR1, HCDR2, HCDR3, LCDR1, LCDR2 and LCDR3 as set forth in SEQ ID NOs 1 - 4, 9 and 6, respectively. Moreover, antibody "NZM var3" comprises the amino acid sequences of HCDR1, HCDR2, HCDR3, LCDR1, LCDR2 and LCDR3 as set forth in SEQ ID NOs 1 - 4, 10 and 6, respectively.

In some embodiments, the antibody, or the antigen binding fragment thereof, comprises the amino acid sequences of HCDR1, HCDR2, HCDR3, LCDR1, LCDR2 and LCDR3 as set forth in SEQ ID NOs 1 - 6, respectively, wherein one or more (e.g., 2, 3, 4, or 5) of the amino acids at positions X₁, X₂, X₃, X₄ and/or X₅ of SEQ ID NO: 5 is a conservative amino acid substitution as compared to SEQ ID NO: 9 (the LCDR2 of "NZM var1") or SEQ ID NO: 10 (the LCDR2 of "NZM var3"). In some embodiments, the antibody, or the antigen binding fragment thereof, comprises the amino acid sequences of HCDR1, HCDR2, HCDR3, LCDR1, LCDR2 and LCDR3 as set forth in SEQ ID NOs 1 - 4, 7 and 6, respectively, wherein one or more (e.g., 2, or 3) of the amino acids at positions X₂, X₃ and/or X₄ of SEQ ID NO: 7 is a conservative amino acid substitution as compared to SEQ ID NO: 9 (the LCDR2 of "NZM var1") or SEQ ID NO: 10 (the LCDR2 of "NZM var3"). Conservative amino acid substitutions are well-known to the skilled person and typically include substitutions, in which the substituted amino acid has similar structural or chemical properties with the corresponding amino acid in the reference sequence. By way of example, conservative amino acid substitutions involve substitution of one aliphatic or hydrophobic amino acid, e.g. alanine, valine, leucine and isoleucine, with another; substitution of one hydoxyl-containing amino acid, e.g. serine and threonine, with another; substitution of one acidic residue, e.g. glutamic acid or aspartic acid, with another; replacement of one amide-containing residue, e.g. asparagine and glutamine, with another; replacement of one aromatic residue, e.g. phenylalanine and tyrosine, with another; replacement of one basic residue, e.g. lysine, arginine and histidine, with another; and/or replacement of one small amino acid, e.g., alanine, serine, threonine, methionine, and glycine, with another.

In some embodiments, the antibody of the invention, or the antigen-binding fragment thereof, comprises a heavy chain variable region (VH) comprising an amino acid sequence having 70% or more (i.e. 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or more) identity to SEQ ID NO: 15 and a light chain variable region comprising the amino acid sequence having 70% or more (i.e. 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or more) identity to SEQ ID NO: 16, in particular wherein the CDR sequences as defined above are maintained.

The same definitions, embodiments and examples as outlined above apply accordingly for the VL sequence of SEQ ID NO: 16, which comprises the LCDR2 as described above. Accordingly, in SEQ ID NO: 16, amino acids X₁, X₂, X₃, X₄ and X₅ are defined as follows:
X₁ may be any amino acid, however, if X₂ is S, X₃ is A, X₄ is L and X₅ is Q, then X₁ is not T;
X₂ may be any amino acid, however, if X₁ is T, X₃ is A, X₄ is L and X₅ is Q, then X₂ is not S;
X₃ may be any amino acid, however, if X₁ is T, X₂ is S, X₄ is L and X₅ is Q, then X₃ is not A;
X₄ may be any amino acid, however, if X₁ is T, X₂ is S, X₃ is A and X₅ is Q, then X₄ is not L; and
X₅ may be any amino acid, however, if X₁ is T, X₂ is S, X₃ is A and X₄ is L, then X₅ is not Q.

Moreover, X₁ of SEQ ID NO: 16 may be T or S. In some embodiments, X₅ of SEQ ID NO: 16 may be Q, A, T or S. In particular, X₁ of SEQ ID NO: 16 may be T and/or X₅ of SEQ ID NO: 16 may be Q.

Preferably, the antibody, or the antigen-binding fragment thereof, comprises the light chain variable region (VH) amino acid sequence of SEQ ID NO: 17 wherein
X₂ may be any amino acid, however, if X₃ is A and X₄ is L, then X₂ is not S;
X₃ may be any amino acid, however, if X₂ is S and X₄ is L, then X₃ is not A; and
X₄ may be any amino acid, however, if X₂ is S and X₃ is A, then X₄ is not L
(SEQ ID NO: 17).

In some embodiments, (i) X₂ in SEQ ID NO: 16 or 17 is selected from G, T, V, K, D and N; and/or (ii) X₄ in SEQ ID NO: 16 or 17 is selected from N, Q, V, I and S. In particular, X₂ in SEQ ID NO: 16 or 17 may be K and/or X₄ in SEQ ID NO: 16 or 17 may be N.

More preferably, the antibody, or the antigen-binding fragment thereof, comprises the light chain variable region (VH) amino acid sequence of SEQ ID NO: 18, wherein X₃ may be any amino acid (SEQ ID NO: 18).

In some embodiments, X₃ in SEQ ID NO: 16 or 17 is selected from A, G, S, T, D, N, V and P. For example, X₃ in SEQ ID NO: 16 or 17 may be selected from A, T, G and S. In particular, X3 in SEQ ID NO: 16 or 17 may be G or S. In some embodiments, the antibody, or the antigen-binding fragment thereof, comprises the VH amino acid sequence of SEQ ID NO: 19. In some embodiments, the antibody, or the antigen-binding fragment thereof, comprises the VH amino acid sequence of SEQ ID NO: 20.

In some embodiments, the antibody, or the antigen binding fragment thereof, comprises the amino acid sequences of VH and VL as set forth in SEQ ID NOs 15 and 16, respectively, wherein one or more (e.g., 2, 3, 4, or 5) of the amino acids at positions X₁, X₂, X₃, X₄ and/or X₅ of SEQ ID NO: 16 is a conservative amino acid substitution as compared to SEQ ID NO: 19 (the VH of "NZM var1") or SEQ ID NO: 20 (the VH of "NZM var3").

In some embodiments, the antibody, or the antigen binding fragment thereof, comprises the amino acid sequences of VH and VL as set forth in SEQ ID NOs 15 and 17, respectively, wherein one or more (e.g., 2, or 3) of the amino acids at positions X₂, X₃ and/or X₄ of SEQ ID NO: 17 is a conservative amino acid substitution as compared to SEQ ID NO: 19 (the VH of "NZM var1") or SEQ ID NO: 20 (the VH of "NZM var3").

Accordingly, the antibody of the invention, or the antigen-binding fragment thereof, may comprise a heavy chain variable region (VH) comprising an amino acid sequence having 70% or more (i.e. 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or more) identity to SEQ ID NO: 15 and a light chain variable region comprising the amino acid sequence having 70% or more (i.e. 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or more) identity to SEQ ID NO: 17, in particular wherein the CDR sequences as defined above are maintained.

Moreover, the antibody of the invention, or the antigen-binding fragment thereof, may comprise a heavy chain variable region (VH) comprising an amino acid sequence having 70% or more (i.e. 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or more) identity to SEQ ID NO: 15 and a light chain variable region comprising the amino acid sequence having 70% or more (i.e. 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or more) identity to SEQ ID NO: 18, in particular wherein the CDR sequences as defined above are maintained.

In particular, the antibody of the invention, or the antigen-binding fragment thereof, may comprise a heavy chain variable region (VH) comprising an amino acid sequence having 70% or more (i.e. 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or more) identity to SEQ ID NO: 15 and a light chain variable region comprising the amino acid sequence having 70% or more (i.e. 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or more) identity to SEQ ID NO: 19 or 20, in particular wherein the CDR sequences as defined above are maintained.

Sequence identity is usually calculated with regard to the full length of the reference sequence (i.e. the sequence recited in the specification). Percentage identity, as referred to herein, can be determined, for example, using BLAST using the default parameters specified by the NCBI (the National Center for Biotechnology Information; http://www.ncbi.nlm.nih.gov/) [Blosum 62 matrix; gap open penalty=11 and gap extension penalty=1].

An "sequence variant" has an altered sequence in which one or more of the amino acids in the reference sequence is/are deleted or substituted, and/or one or more amino acids is/are inserted into the sequence of the reference amino acid sequence. As a result of the alterations, the amino acid sequence variant has an amino acid sequence which is, for example, at least 70% identical to the reference sequence. Variant sequences which are, for example, at least 70% identical have no more than 30 alterations, i.e. any combination of deletions, insertions or substitutions, per 100 amino acids of the reference sequence.

In some embodiments, the antibody of the invention, or the antigen-binding fragment thereof, comprises a heavy chain variable region comprising an amino acid sequence having 75% or more (i.e. 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or more) identity to SEQ ID NO: 15 and a light chain variable region comprising the amino acid sequence having 75% or more (i.e. 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or more) identity to SEQ ID NO: 16, in particular wherein the CDR sequences as defined above are maintained. In some embodiments, the antibody of the invention, or the antigen-binding fragment thereof, comprises a heavy chain variable region comprising an amino acid sequence having 75% or more (i.e. 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or more) identity to SEQ ID NO: 15 and a light chain variable region comprising the amino acid sequence having 75% or more (i.e. 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or more) identity to SEQ ID NO: 17, in particular wherein the CDR sequences as defined above are maintained. In some embodiments, the antibody of the invention, or the antigen-binding fragment thereof, comprises a heavy chain variable region comprising an amino acid sequence having 75% or more (i.e. 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or more) identity to SEQ ID NO: 15 and a light chain variable region comprising the amino acid sequence having 75% or more (i.e. 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or more) identity to SEQ ID NO: 18, in particular wherein the CDR sequences as defined above are maintained. In some embodiments, the antibody of the invention, or the antigen-binding fragment thereof, comprises a heavy chain variable region comprising an amino acid sequence having 75% or more (i.e. 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or more) identity to SEQ ID NO: 15 and a light chain variable region comprising the amino acid sequence having 75% or more (i.e. 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or more) identity to SEQ ID NO: 19, in particular wherein the CDR sequences as defined above are maintained. In some embodiments, the antibody of the invention, or the antigen-binding fragment thereof, comprises a heavy chain variable region comprising an amino acid sequence having 75% or more (i.e. 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or more) identity to SEQ ID NO: 15 and a light chain variable region comprising the amino acid sequence having 75% or more (i.e. 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or more) identity to SEQ ID NO: 20, in particular wherein the CDR sequences as defined above are maintained.

In some embodiments, the antibody of the invention, or the antigen-binding fragment thereof, comprises a heavy chain variable region comprising an amino acid sequence having 80% or more (i.e. 81%, 82%, 83%, 84%, 85%, 86%, 87%, 88%, 89%, 90%, 91%, 92%, 93%, 94%, 95%, 96%, 97%, 98%, 99% or more) identity to SEQ ID NO: 15 and a light chain variable region comprising the amino acid sequence having 80% or more (i.e. 81%, 82%, 83%, 84%, 85%, 86%, 87%, 88%, 89%, 90%, 91%, 92%, 93%, 94%, 95%, 96%, 97%, 98%, 99% or more) identity to SEQ ID NO: 16, in particular wherein the CDR sequences as defined above are maintained. In some embodiments, the antibody of the invention, or the antigen-binding fragment thereof, comprises a heavy chain variable region comprising an amino acid sequence having 80% or more (i.e. 81%, 82%, 83%, 84%, 85%, 86%, 87%, 88%, 89%, 90%, 91%, 92%, 93%, 94%, 95%, 96%, 97%, 98%, 99% or more) identity to SEQ ID NO: 15 and a light chain variable region comprising the amino acid sequence having 80% or more (i.e. 81%, 82%, 83%, 84%, 85%, 86%, 87%, 88%, 89%, 90%, 91%, 92%, 93%, 94%, 95%, 96%, 97%, 98%, 99% or more) identity to SEQ ID NO: 17, in particular wherein the CDR sequences as defined above are maintained. In some embodiments, the antibody of the invention, or the antigen-binding fragment thereof, comprises a heavy chain variable region comprising an amino acid sequence having 80% or more (i.e. 81%, 82%, 83%, 84%, 85%, 86%, 87%, 88%, 89%, 90%, 91%, 92%, 93%, 94%, 95%, 96%, 97%, 98%, 99% or more) identity to SEQ ID NO: 15 and a light chain variable region comprising the amino acid sequence having 80% or more (i.e. 81%, 82%, 83%, 84%, 85%, 86%, 87%, 88%, 89%, 90%, 91%, 92%, 93%, 94%, 95%, 96%, 97%, 98%, 99% or more) identity to SEQ ID NO: 18, in particular wherein the CDR sequences as defined above are maintained. In some embodiments, the antibody of the invention, or the antigen-binding fragment thereof, comprises a heavy chain variable region comprising an amino acid sequence having 80% or more (i.e. 81%, 82%, 83%, 84%, 85%, 86%, 87%, 88%, 89%, 90%, 91%, 92%, 93%, 94%, 95%, 96%, 97%, 98%, 99% or more) identity to SEQ ID NO: 15 and a light chain variable region comprising the amino acid sequence having 80% or more (i.e. 81%, 82%, 83%, 84%, 85%, 86%, 87%, 88%, 89%, 90%, 91%, 92%, 93%, 94%, 95%, 96%, 97%, 98%, 99% or more) identity to SEQ ID NO: 19, in particular wherein the CDR sequences as defined above are maintained. In some embodiments, the antibody of the invention, or the antigen-binding fragment thereof, comprises a heavy chain variable region comprising an amino acid sequence having 80% or more (i.e. 81%, 82%, 83%, 84%, 85%, 86%, 87%, 88%, 89%, 90%, 91%, 92%, 93%, 94%, 95%, 96%, 97%, 98%, 99% or more) identity to SEQ ID NO: 15 and a light chain variable region comprising the amino acid sequence having 80% or more (i.e. 81%, 82%, 83%, 84%, 85%, 86%, 87%, 88%, 89%, 90%, 91%, 92%, 93%, 94%, 95%, 96%, 97%, 98%, 99% or more) identity to SEQ ID NO: 20, in particular wherein the CDR sequences as defined above are maintained.

In some embodiments, the antibody of the invention, or the antigen-binding fragment thereof, comprises a heavy chain variable region comprising an amino acid sequence having 85% or more (i.e. 86%, 87%, 88%, 89%, 90%, 91%, 92%, 93%, 94%, 95%, 96%, 97%, 98%, 99% or more) identity to SEQ ID NO: 15 and a light chain variable region comprising the amino acid sequence having 85% or more (i.e. 86%, 87%, 88%, 89%, 90%, 91%, 92%, 93%, 94%, 95%, 96%, 97%, 98%, 99% or more) identity to SEQ ID NO: 16, in particular wherein the CDR sequences as defined above are maintained. In some embodiments, the antibody of the invention, or the antigen-binding fragment thereof, comprises a heavy chain variable region comprising an amino acid sequence having 85% or more (i.e. 86%, 87%, 88%, 89%, 90%, 91%, 92%, 93%, 94%, 95%, 96%, 97%, 98%, 99% or more) identity to SEQ ID NO: 15 and a light chain variable region comprising the amino acid sequence having 85% or more (i.e. 86%, 87%, 88%, 89%, 90%, 91%, 92%, 93%, 94%, 95%, 96%, 97%, 98%, 99% or more) identity to SEQ ID NO: 17, in particular wherein the CDR sequences as defined above are maintained. In some embodiments, the antibody of the invention, or the antigen-binding fragment thereof, comprises a heavy chain variable region comprising an amino acid sequence having 85% or more (i.e. 86%, 87%, 88%, 89%, 90%, 91%, 92%, 93%, 94%, 95%, 96%, 97%, 98%, 99% or more) identity to SEQ ID NO: 15 and a light chain variable region comprising the amino acid sequence having 85% or more (i.e. 86%, 87%, 88%, 89%, 90%, 91%, 92%, 93%, 94%, 95%, 96%, 97%, 98%, 99% or more) identity to SEQ ID NO: 18, in particular wherein the CDR sequences as defined above are maintained. In some embodiments, the antibody of the invention, or the antigen-binding fragment thereof, comprises a heavy chain variable region comprising an amino acid sequence having 85% or more (i.e. 86%, 87%, 88%, 89%, 90%, 91%, 92%, 93%, 94%, 95%, 96%, 97%, 98%, 99% or more) identity to SEQ ID NO: 15 and a light chain variable region comprising the amino acid sequence having 85% or more (i.e. 86%, 87%, 88%, 89%, 90%, 91%, 92%, 93%, 94%, 95%, 96%, 97%, 98%, 99% or more) identity to SEQ ID NO: 19, in particular wherein the CDR sequences as defined above are maintained. In some embodiments, the antibody of the invention, or the antigen-binding fragment thereof, comprises a heavy chain variable region comprising an amino acid sequence having 85% or more (i.e. 86%, 87%, 88%, 89%, 90%, 91%, 92%, 93%, 94%, 95%, 96%, 97%, 98%, 99% or more) identity to SEQ ID NO: 15 and a light chain variable region comprising the amino acid sequence having 85% or more (i.e. 86%, 87%, 88%, 89%, 90%, 91%, 92%, 93%, 94%, 95%, 96%, 97%, 98%, 99% or more) identity to SEQ ID NO: 20, in particular wherein the CDR sequences as defined above are maintained.

In some embodiments, the antibody of the invention, or the antigen-binding fragment thereof, comprises a heavy chain variable region comprising an amino acid sequence having 90% or more (i.e. 91%, 92%, 93%, 94%, 95%, 96%, 97%, 98%, 99% or more) identity to SEQ ID NO: 15 and a light chain variable region comprising the amino acid sequence having 90% or more (i.e. 91%, 92%, 93%, 94%, 95%, 96%, 97%, 98%, 99% or more) identity to SEQ ID NO: 16, in particular wherein the CDR sequences as defined above are maintained. In some embodiments, the antibody of the invention, or the antigen-binding fragment thereof, comprises a heavy chain variable region comprising an amino acid sequence having 90% or more (i.e. 91%, 92%, 93%, 94%, 95%, 96%, 97%, 98%, 99% or more) identity to SEQ ID NO: 15 and a light chain variable region comprising the amino acid sequence having 90% or more (i.e. 91%, 92%, 93%, 94%, 95%, 96%, 97%, 98%, 99% or more) identity to SEQ ID NO: 17, in particular wherein the CDR sequences as defined above are maintained. In some embodiments, the antibody of the invention, or the antigen-binding fragment thereof, comprises a heavy chain variable region comprising an amino acid sequence having 90% or more (i.e. 91%, 92%, 93%, 94%, 95%, 96%, 97%, 98%, 99% or more) identity to SEQ ID NO: 15 and a light chain variable region comprising the amino acid sequence having 90% or more (i.e. 91%, 92%, 93%, 94%, 95%, 96%, 97%, 98%, 99% or more) identity to SEQ ID NO: 18, in particular wherein the CDR sequences as defined above are maintained. In some embodiments, the antibody of the invention, or the antigen-binding fragment thereof, comprises a heavy chain variable region comprising an amino acid sequence having 90% or more (i.e. 91%, 92%, 93%, 94%, 95%, 96%, 97%, 98%, 99% or more) identity to SEQ ID NO: 15 and a light chain variable region comprising the amino acid sequence having 90% or more (i.e. 91%, 92%, 93%, 94%, 95%, 96%, 97%, 98%, 99% or more) identity to SEQ ID NO: 19, in particular wherein the CDR sequences as defined above are maintained. In some embodiments, the antibody of the invention, or the antigen-binding fragment thereof, comprises a heavy chain variable region comprising an amino acid sequence having 90% or more (i.e. 91%, 92%, 93%, 94%, 95%, 96%, 97%, 98%, 99% or more) identity to SEQ ID NO: 15 and a light chain variable region comprising the amino acid sequence having 90% or more (i.e. 91%, 92%, 93%, 94%, 95%, 96%, 97%, 98%, 99% or more) identity to SEQ ID NO: 20, in particular wherein the CDR sequences as defined above are maintained.

In some embodiments, the antibody of the invention, or the antigen-binding fragment thereof, comprises a heavy chain variable region comprising an amino acid sequence having 95% or more (i.e. 96%, 97%, 98%, 99% or more) identity to SEQ ID NO: 15 and a light chain variable region comprising the amino acid sequence having 95% or more (i.e. 96%, 97%, 98%, 99% or more) identity to SEQ ID NO: 16, in particular wherein the CDR sequences as defined above are maintained. In some embodiments, the antibody of the invention, or the antigen-binding fragment thereof, comprises a heavy chain variable region comprising an amino acid sequence having 95% or more (i.e. 96%, 97%, 98%, 99% or more) identity to SEQ ID NO: 15 and a light chain variable region comprising the amino acid sequence having 95% or more (i.e. 96%, 97%, 98%, 99% or more) identity to SEQ ID NO: 17, in particular wherein the CDR sequences as defined above are maintained. In some embodiments, the antibody of the invention, or the antigen-binding fragment thereof, comprises a heavy chain variable region comprising an amino acid sequence having 95% or more (i.e. 96%, 97%, 98%, 99% or more) identity to SEQ ID NO: 15 and a light chain variable region comprising the amino acid sequence having 95% or more (i.e. 96%, 97%, 98%, 99% or more) identity to SEQ ID NO: 18, in particular wherein the CDR sequences as defined above are maintained. In some embodiments, the antibody of the invention, or the antigen-binding fragment thereof, comprises a heavy chain variable region comprising an amino acid sequence having 95% or more (i.e. 96%, 97%, 98%, 99% or more) identity to SEQ ID NO: 15 and a light chain variable region comprising the amino acid sequence having 95% or more (i.e. 96%, 97%, 98%, 99% or more) identity to SEQ ID NO: 19, in particular wherein the CDR sequences as defined above are maintained. In some embodiments, the antibody of the invention, or the antigen-binding fragment thereof, comprises a heavy chain variable region comprising an amino acid sequence having 95% or more (i.e. 96%, 97%, 98%, 99% or more) identity to SEQ ID NO: 15 and a light chain variable region comprising the amino acid sequence having 95% or more (i.e. 96%, 97%, 98%, 99% or more) identity to SEQ ID NO: 20, in particular wherein the CDR sequences as defined above are maintained.

In some embodiments, the antibody, or the antigen-binding fragment thereof, comprises a heavy chain variable region comprising an amino acid sequence as set forth in SEQ ID NO: 15 and a light chain variable region comprising the amino acid sequence as set forth in SEQ ID NO: 16.

Preferably, the antibody, or the antigen-binding fragment thereof, comprises a heavy chain variable region comprising an amino acid sequence as set forth in SEQ ID NO: 15 and a light chain variable region comprising the amino acid sequence as set forth in SEQ ID NO: 17.

More preferably, the antibody, or the antigen-binding fragment thereof, comprises a heavy chain variable region comprising an amino acid sequence as set forth in SEQ ID NO: 15 and a light chain variable region comprising the amino acid sequence as set forth in SEQ ID NO: 18.

Even more preferably, the antibody, or the antigen-binding fragment thereof, comprises a heavy chain variable region comprising an amino acid sequence as set forth in SEQ ID NO: 15 and a light chain variable region comprising the amino acid sequence as set forth in SEQ ID NO: 19 or 20.

In particular, the antibody of the invention, or an antigen-binding fragment thereof, binds (specifically) to α4 integrin. Accordingly, the antibody of the invention, or an antigen-binding fragment thereof, maintains the functionality of NZM. In other words, the antibody, or the antigen binding fragment thereof, binds to the same target as NZM.

Standard methods to assess binding of the antibody according to the present invention, or the antigen-binding fragment thereof, are known to those skilled in the art and include, for example, ELISA (enzyme-linked immunosorbent assay) and FACS analysis. An exemplified method is described in Example 3. Briefly, serial dilutions of test and control antibodies may be prepared. For binding to α4 integrin, T cells isolated from healthy donors may be used as source of α4-integrin and added to the plates. T cells may then be washed and stained with a labelled anti-human IgG antibody, such as Alexa Fluor 647-conjugated goat anti-human IgG. Cells may then be washed and analyzed by FACS. NZM binding can be calculated as percentage of IgG⁺ stained cells. In general, binding of an antibody of the invention to α4 integrin may be compared to that of NZM to α4 integrin in the same assay in order to determine whether or not the functionality of NZM is maintained.

In some embodiments, the binding affinity of the antibody, or the antigen-binding fragment thereof, to an MHC molecule is decreased in comparison to the binding affinity of natalizumab to the same MHC molecule. Similarly as for binding to α4 integrin, the skilled person is also aware of methods to determine binding to MHC molecules. In general, it is understood that for comparative purposes the test antibody is usually compared with NZM in the same approach, i.e. under the same conditions and regarding the same MHC molecule. In this way, the skilled person can easily determine whether or not the binding affinity of the antibody, or the antigen-binding fragment thereof, to an MHC molecule is decreased in comparison to the binding affinity of natalizumab to the same MHC molecule. Preferably, the MHC molecule is an MHC II molecule. More preferably, the MHC molecule is a HLA-DRB1*13 and/or HLA-DRB1*14 allele of MHC II. DRB1*13/14 that were found to be associated to NZM-related allergic reactions (de la Hera, B., et al. (2014) Natalizumab-related anaphylactoid reactions in MS patients are associated with HLA class II alleles. Neurol Neuroimmunol Neuroinflamm 1, e47). In particular, this study (de la Hera et al., 2014) reported a positive association of NZM-related allergic reactions with DRB1*13 and 14 alleles, suggesting a critical role for presentation of NZM peptides on MHC class II molecules and T-cell dependent B cell activation for the production of ADAs to NZM.

For example, the binding affinity to MHC molecules may be determined *in silico* (e.g., as described in Examples 1 and 4). Briefly, NetMHCIIpan 3.2 server (Jensen KK, Andreatta M, Marcatili P, Buus S, Greenbaum JA, Yan Z, Sette A, Peters B, Nielsen M.: Improved methods for predicting peptide binding affinity to MHC class II molecules. Immunology. 2018 Jan 6. doi: 10.1111/imm.12889; URL: http://www.cbs.dtu.dk/services/NetMHCIIpan-3.2/) may be used to predict the IC50 (nM) values (e.g., of all theoretical antibody-derived 15mer peptides) binding to a reference set of nine HLA-DRB1 and HLA-DRB3/4/5 alleles (including DRB1*03:01, DRB1*07:01, DRB1*13:01, DRB1*14:01, DRB1*15:01, DRB3*01:01, DRB3*02:02, DRB4*01:01, and DRB5*01:01) (as described in Paul, S., et al. Development and validation of a broad scheme for prediction of HLA class II restricted T cell epitopes. J Immunol Methods 422, 28-34 (2015), which is incorporated herein by reference).

In some embodiments, the antibody of the invention is a humanized antibody. In some embodiments, the antibody of the invention is a monoclonal antibody. In some embodiments, the antibody of the invention is a purified antibody. In some embodiments, the antibody of the invention is a single chain antibody. In some embodiments, the antibody of the invention is a Fab, Fab', F(ab')2, Fv or scFv.

Antibodies of the invention can be of any isotype (*e.g*., IgA, IgG, IgM i.e. an α, γ or µ heavy chain). For example, the antibody is of the IgG type. Within the IgG isotype, antibodies may be IgG1, IgG2, IgG3 or IgG4 subclass, for example IgG4. Antibodies of the invention may have a κ or a λ light chain. In some embodiments, the antibody is of IgG4 type and has a lambda or kappa light chain.

In some embodiments, the antibody according to the present invention, or an antigen binding fragment thereof, comprises an Fc moiety. The Fc moiety may be derived from human origin, e.g. from human IgG1, IgG2, IgG3, and/or IgG4, such as human IgG4.

As used herein, the term "Fc moiety" refers to a sequence derived from the portion of an immunoglobulin heavy chain beginning in the hinge region just upstream of the papain cleavage site (e.g., residue 216 in native IgG, taking the first residue of heavy chain constant region to be 114) and ending at the C-terminus of the immunoglobulin heavy chain. Accordingly, an Fc moiety may be a complete Fc moiety or a portion (e.g., a domain) thereof. A complete Fc moiety comprises at least a hinge domain, a CH2 domain, and a CH3 domain (e.g., EU amino acid positions 216-446). An additional lysine residue (K) is sometimes present at the extreme C-terminus of the Fc moiety, but is often cleaved from a mature antibody.

Each of the amino acid positions within an Fc moiety have been numbered herein according to the art-recognized EU numbering system of Kabat, see e.g., by Kabat et al., in "Sequences of Proteins of Immunological Interest", U.S. Dept. Health and Human Services, 1983 and 1987. The EU index or EU index as in Kabat or EU numbering refers to the numbering of the EU antibody (Edelman GM, Cunningham BA, Gall WE, Gottlieb PD, Rutishauser U, Waxdal MJ. The covalent structure of an entire gammaG immunoglobulin molecule. Proc Natl Acad Sci USA. 1969;63(1):78-85; Kabat E.A., National Institutes of Health (U.S.) Office of the Director, "Sequences of Proteins of Immunological Interest", 5th edition, Bethesda, MD : U.S. Dept. of Health and Human Services, Public Health Service, National Institutes of Health, 1991, hereby entirely incorporated by reference).

In some embodiments, in the context of the present invention an Fc moiety comprises at least one of: a hinge (e.g., upper, middle, and/or lower hinge region) domain, a CH2 domain, a CH3 domain, or a variant, portion, or fragment thereof. An Fc moiety may comprise at least a hinge domain, a CH2 domain or a CH3 domain. The Fc moiety may be a complete Fc moiety. The Fc moiety may also comprises one or more amino acid insertions, deletions, or substitutions relative to a naturally-occurring Fc moiety. For example, at least one of a hinge domain, CH2 domain or CH3 domain (or portion thereof) may be deleted. For example, an Fc moiety may comprise or consist of: (i) hinge domain (or portion thereof) fused to a CH2 domain (or portion thereof), (ii) a hinge domain (or portion thereof) fused to a CH3 domain (or portion thereof), (iii) a CH2 domain (or portion thereof) fused to a CH3 domain (or portion thereof), (iv) a hinge domain (or portion thereof), (v) a CH2 domain (or portion thereof), or (vi) a CH3 domain or portion thereof.

It will be understood by one of ordinary skill in the art that the Fc moiety may be modified such that it varies in amino acid sequence from the complete Fc moiety of a naturally occurring immunoglobulin molecule, while retaining at least one desirable function conferred by the naturally-occurring Fc moiety. Such functions include Fc receptor (FcR) binding, antibody half-life modulation, ADCC function, protein A binding, protein G binding, and complement binding. The portions of naturally occurring Fc moieties, which are responsible and/or essential for such functions are well known by those skilled in the art.

For example, to activate the complement cascade C1q binds to at least two molecules of IgG or one molecule of IgM, attached to the antigenic target (Ward, E. S., and Ghetie, V., Ther. Immunol. 2 (1995) 77-94). Burton, D. R., described (Mol. Immunol. 22 (1985) 161-206) that the heavy chain region comprising amino acid residues 318 to 337 is involved in complement fixation. Duncan, A. R., and Winter, G. (Nature 332 (1988) 738-740), using site directed mutagenesis, reported that Glu318, Lys320 and Lys322 form the binding site to C1q. The role of Glu318, Lys320 and Lys 322 residues in the binding of C1q was confirmed by the ability of a short synthetic peptide containing these residues to inhibit complement mediated lysis.

For example, FcR binding can be mediated by the interaction of the Fc moiety (of an antibody) with Fc receptors (FcRs), which are specialized cell surface receptors on hematopoietic cells. Fc receptors belong to the immunoglobulin superfamily, and were shown to mediate both the removal of antibody-coated pathogens by phagocytosis of immune complexes, and the lysis of erythrocytes and various other cellular targets (e.g. tumor cells) coated with the corresponding antibody, via antibody dependent cell mediated cytotoxicity (ADCC; Van de Winkel, J. G., and Anderson, C. L., J. Leukoc. Bio/. 49 (1991) 511-524). FcRs are defined by their specificity for immunoglobulin classes; Fc receptors for IgG antibodies are referred to as FcγR, for IgE as FcεR, for IgA as FcαR and so on and neonatal Fc receptors are referred to as FcRn. Fc receptor binding is described for example in Ravetch, J. V., and Kinet, J. P., Annu. Rev. Immunol. 9 (1991) 457-492; Capel, P. J., et al., Immunomethods 4 (1994) 25-34; de Haas, M., et al., J Lab. Clin. Med. 126 (1995) 330-341; and Gessner, J. E., et al., Ann. Hematol. 76 (1998) 231-248.

Cross-linking of receptors by the Fc domain of native IgG antibodies (FcγR) triggers a wide variety of effector functions including phagocytosis, antibody-dependent cellular cytotoxicity, and release of inflammatory mediators, as well as immune complex clearance and regulation of antibody production. Therefore, the Fc moiety may provide cross-linking of receptors (FcγR). In humans, three classes of FcγR have been characterized, which are: (i) FcγRI (CD64), which binds monomeric IgG with high affinity and is expressed on macrophages, monocytes, neutrophils and eosinophils; (ii) FcγRII (CD32), which binds complexed IgG with medium to low affinity, is widely expressed, in particular on leukocytes, is known to be a central player in antibody-mediated immunity, and which can be divided into FcγRIIA, FcγRIIB and FcγRIIC, which perform different functions in the immune system, but bind with similar low affinity to the IgG-Fc, and the ectodomains of these receptors are highly homologuous; and (iii) FcγRIII (CD16), which binds IgG with medium to low affinity and exists as two types: FcγRIIIA found on NK cells, macrophages, eosinophils and some monocytes and T cells and mediating ADCC and FcγRIIIB, which is highly expressed on neutrophils. FcγRIIA is found on many cells involved in killing (e.g. macrophages, monocytes, neutrophils) and seems able to activate the killing process. FcγRIIB seems to play a role in inhibitory processes and is found on B-cells, macrophages and on mast cells and eosinophils. Importantly, 75% of all FcγRIIB is found in the liver (Ganesan, L. P. et al., 2012: FcγRIIb on liver sinusoidal endothelium clears small immune complexes. Journal of Immunology 189: 4981-4988). FcγRIIB is abundantly expressed on Liver Sinusoidal Endothelium, called LSEC, and in Kupffer cells in the liver and LSEC are the major site of small immune complexes clearance (Ganesan, L. P. et al., 2012: FcγRIIb on liver sinusoidal endothelium clears small immune complexes. Journal of Immunology 189: 4981-4988).

Accordingly, antibodies, and antigen binding fragments thereof, of the invention may be able to bind to FcγRIIb, for example antibodies comprising an Fc moiety for binding to FcγRIIb, in particular an Fc region, such as, for example IgG-type antibodies. Moreover, it is possible to engineer the Fc moiety to enhance FcγRIIB binding by introducing the mutations S267E and L328F as described by Chu, S. Y. et al., 2008: Inhibition of B cell receptor-mediated activation of primary human B cells by coengagement of CD19 and FcγRIIb with Fc-engineered antibodies. Molecular Immunology 45, 3926-3933. Thereby, the clearance of immune complexes can be enhanced (Chu, S., et al., 2014: Accelerated Clearance of IgE In Chimpanzees Is Mediated By Xmab7195, An Fc-Engineered Antibody With Enhanced Affinity For Inhibitory Receptor FcγRIIb. Am J Respir Crit, American Thoracic Society International Conference Abstracts). Accordingly, the antibodies, or antigen binding fragments thereof, of the invention may comprise an engineered Fc moiety with the mutations S267E and L328F, in particular as described by Chu, S. Y. et al., 2008: Inhibition of B cell receptor-mediated activation of primary human B cells by coengagement of CD19 and FcγRIIb with Fc-engineered antibodies. Molecular Immunology 45, 3926-3933.

On B-cells it seems to function to suppress further immunoglobulin production and isotype switching to say for example the IgE class. On macrophages, FcγRIIB acts to inhibit phagocytosis as mediated through FcγRIIA. On eosinophils and mast cells the b form may help to suppress activation of these cells through IgE binding to its separate receptor.

Regarding FcγRI binding, modification in native IgG of at least one of E233-G236, P238, D265, N297, A327 and P329 reduces binding to FcγRI. IgG2 residues at positions 233-236, substituted into IgG1 and IgG4, reduces binding to FcγRI by 10³-fold and eliminated the human monocyte response to antibody-sensitized red blood cells (Armour, K. L., et al. Eur. J. Immunol. 29 (1999) 2613-2624). Regarding FcγRII binding, reduced binding for FcγRIIA is found e.g. for IgG mutation of at least one of E233-G236, P238, D265, N297, A327, P329, D270, Q295, A327, R292 and K414. Regarding FcγRIII binding, reduced binding to FcγRIIIA is found e.g. for mutation of at least one of E233-G236, P238, D265, N297, A327, P329, D270, Q295, A327, S239, E269, E293, Y296, V303, A327, K338 and D376. Mapping of the binding sites on human IgG1 for Fc receptors, the above mentioned mutation sites and methods for measuring binding to FcγRI and FcγRIIA are described in Shields, R. L., et al., J. Biol. Chem. 276 (2001) 6591-6604. For example, a single (S239D or I332E), double (S239D/I332E), and triple mutations (S239D/I332E/A330L) improved the affinity against human FcγRIIIa. Furthermore, the addition of the mutation G236A to S239D/I332E improved not only FcγRIIa:FcγRIIb ratio, but also enhanced binding to FcγRIIIa. Accordingly, the mutations G236A/S239D/A330L/I332E were described to enhance engagement of FcγRIIa and FcγRIIIa.

Regarding binding to FcγRII, two regions of native IgG Fc appear to be critical for interactions of FcγRIIs and IgGs, namely (i) the lower hinge site of IgG Fc, in particular amino acid residues L, L, G, G (234 - 237, EU numbering), and (ii) the adjacent region of the CH2 domain of IgG Fc, in particular a loop and strands in the upper CH2 domain adjacent to the lower hinge region, e.g. in a region of P331 (Wines, B.D., et al., J. Immunol. 2000; 164: 5313 - 5318). Moreover, FcγRI appears to bind to the same site on IgG Fc, whereas FcRn and Protein A bind to a different site on IgG Fc, which appears to be at the CH2-CH3 interface (Wines, B.D., et al., J. Immunol. 2000; 164: 5313 - 5318).

For example, the Fc moiety may comprise or consist of at least the portion of an Fc moiety that is known in the art to be required for FcRn binding or extended half-life. Alternatively or additionally, the Fc moiety of the antibody of the invention comprises at least the portion of known in the art to be required for Protein A binding and/or the Fc moiety of the antibody of the invention comprises at least the portion of an Fc molecule known in the art to be required for protein G binding. The Fc moiety may comprise at least the portion known in the art to be required for FcγR binding. As outlined above, an Fc moiety may thus at least comprise (i) the lower hinge site of native IgG Fc, in particular amino acid residues L, L, G, G (234 - 237, EU numbering), and (ii) the adjacent region of the CH2 domain of native IgG Fc, in particular a loop and strands in the upper CH2 domain adjacent to the lower hinge region, e.g. in a region of P331, for example a region of at least 3, 4, 5, 6, 7, 8, 9, or 10 consecutive amino acids in the upper CH2 domain of native IgG Fc around P331, e.g. between amino acids 320 and 340 (EU numbering) of native IgG Fc.

In some embodiments, the antibody, or antigen binding fragment thereof, according to the present invention comprises an Fc region. As used herein, the term "Fc region" refers to the portion of an immunoglobulin formed by two or more Fc moieties of antibody heavy chains. For example, the Fc region may be monomeric or "single-chain" Fc region (i.e., a scFc region). Single chain Fc regions are comprised of Fc moieties linked within a single polypeptide chain (e.g., encoded in a single contiguous nucleic acid sequence). Exemplary scFc regions are disclosed in WO 2008/143954 A2. The Fc region may be dimeric. A "dimeric Fc region" or "dcFc" refers to the dimer formed by the Fc moieties of two separate immunoglobulin heavy chains. The dimeric Fc region may be a homodimer of two identical Fc moieties (e.g., an Fc region of a naturally occurring immunoglobulin) or a heterodimer of two non-identical Fc moieties.

The Fc moieties of the Fc region may be of the same or different class and/or subclass. For example, the Fc moieties may be derived from an immunoglobulin (e.g., a human immunoglobulin) of an IgG1, IgG2, IgG3 or IgG4 subclass. The Fc moieties of the Fc region may be of the same class and subclass. However, the Fc region (or one or more Fc moieties of an Fc region) may also be chimeric, whereby a chimeric Fc region may comprise Fc moieties derived from different immunoglobulin classes and/or subclasses. For example, at least two of the Fc moieties of a dimeric or single-chain Fc region may be from different immunoglobulin classes and/or subclasses. Additionally or alternatively, the chimeric Fc regions may comprise one or more chimeric Fc moieties. For example, the chimeric Fc region or moiety may comprise one or more portions derived from an immunoglobulin of a first subclass (e.g., an IgG1, IgG2, IgG3 or IgG4 subclass) while the remainder of the Fc region or moiety is of a different subclass. For example, an Fc region or moiety of an Fc polypeptide may comprise a CH2 and/or CH3 domain derived from an immunoglobulin of a first subclass (e.g., an IgG1, IgG2 or IgG4 subclass) and a hinge region from an immunoglobulin of a second subclass (e.g., an IgG3 subclass). For example, the Fc region or moiety may comprise a hinge and/or CH2 domain derived from an immunoglobulin of a first subclass (e.g., an IgG4 subclass) and a CH3 domain from an immunoglobulin of a second subclass (e.g., an IgG1, IgG2, or IgG3 subclass). For example, the chimeric Fc region may comprise an Fc moiety (e.g., a complete Fc moiety) from an immunoglobulin for a first subclass (e.g., an IgG4 subclass) and an Fc moiety from an immunoglobulin of a second subclass (e.g., an IgG1, IgG2 or IgG3 subclass). For example, the Fc region or moiety may comprise a CH2 domain from an IgG4 immunoglobulin and a CH3 domain from an IgG1 immunoglobulin. For example, the Fc region or moiety may comprise a CH1 domain and a CH2 domain from an IgG4 molecule and a CH3 domain from an IgG1 molecule. For example, the Fc region or moiety may comprise a portion of a CH2 domain from a particular subclass of antibody, e.g., EU positions 292-340 of a CH2 domain. For example, an Fc region or moiety may comprise amino acids a positions 292-340 of CH2 derived from an IgG4 moiety and the remainder of CH2 derived from an IgG1 moiety (alternatively, 292-340 of CH2 may be derived from an IgG1 moiety and the remainder of CH2 derived from an IgG4 moiety).

Moreover, an Fc region or moiety may (additionally or alternatively) for example comprise a chimeric hinge region. For example, the chimeric hinge may be derived, e.g. in part, from an IgG1, IgG2, or IgG4 molecule (e.g., an upper and lower middle hinge sequence) and, in part, from an IgG3 molecule (e.g., an middle hinge sequence). In another example, an Fc region or moiety may comprise a chimeric hinge derived, in part, from an IgG1 molecule and, in part, from an IgG4 molecule. In another example, the chimeric hinge may comprise upper and lower hinge domains from an IgG4 molecule and a middle hinge domain from an IgG1 molecule. Such a chimeric hinge may be made, for example, by introducing a proline substitution (Ser228Pro) at EU position 228 in the middle hinge domain of an IgG4 hinge region. In other embodiments, the chimeric hinge can comprise amino acids at EU positions 233-236 are from an IgG2 antibody and/or the Ser228Pro mutation, wherein the remaining amino acids of the hinge are from an IgG4 antibody (e.g., a chimeric hinge of the sequence ESKYGPPCPPCPAPPVAGP). Further chimeric hinges, which may be used in the Fc moiety of the antibody according to the present invention are described in US 2005/0163783 A1.

In some embodiments, the Fc moiety, or the Fc region, comprises or consists of an amino acid sequence derived from a human immunoglobulin sequence (e.g., from an Fc region or Fc moiety from a human IgG molecule). However, polypeptides may comprise one or more amino acids from another mammalian species. For example, a primate Fc moiety or a primate binding site may be included in the subject polypeptides. Alternatively, one or more murine amino acids may be present in the Fc moiety or in the Fc region.

In some embodiments, the antibody according to the present invention comprises, in particular in addition to an Fc moiety as described above, other parts derived from a constant region, in particular from a constant region of IgG, such as a constant region of (human) IgG4. The antibody according to the present invention may comprise, in particular in addition to an Fc moiety as described above, all other parts of the constant regions, in particular all other parts of the constant regions of IgG (such as (human) IgG4).

Example sequences of constant regions are the amino acid sequences according to SEQ ID NOs: 35 and 36. For example, the amino acid sequence of IgG4 CH1-CH2-CH3 is according to SEQ ID NO: 35 or a sequence variant thereof (including, for example, 1, 2, 3, 4, 5, 6, 7, 8, 9, 10 or more mutations) having at least 70%, at least 75%, at least 80%, at least 85%, at least 90%, at least 95% or at least 99% sequence identity.

For example, the antibody of the invention, or the antigen-binding fragment thereof, may comprise a heavy chain comprising an amino acid sequence having 70% or more (i.e. 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or more) identity to SEQ ID NO: 25 and a light chain comprising the amino acid sequence having 70% or more (i.e. 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or more) identity to SEQ ID NO: 26, in particular wherein the CDR sequences as defined above are maintained. In some embodiments, the antibody, or the antigen-binding fragment thereof, comprises a heavy chain comprising an amino acid sequence as set forth in SEQ ID NO: 25 and a light chain comprising the amino acid sequence as set forth in SEQ ID NO: 26.

In some embodiments, the antibody of the invention, or the antigen-binding fragment thereof, may comprise a heavy chain comprising an amino acid sequence having 70% or more (i.e. 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or more) identity to SEQ ID NO: 25 and a light chain comprising the amino acid sequence having 70% or more (i.e. 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or more) identity to SEQ ID NO: 27, in particular wherein the CDR sequences as defined above are maintained. In some embodiments, the antibody, or the antigen-binding fragment thereof, comprises a heavy chain comprising an amino acid sequence as set forth in SEQ ID NO: 25 and a light chain comprising the amino acid sequence as set forth in SEQ ID NO: 27.

Preferably, the antibody of the invention, or the antigen-binding fragment thereof, may comprise a heavy chain comprising an amino acid sequence having 70% or more (i.e. 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or more) identity to SEQ ID NO: 25 and a light chain comprising the amino acid sequence having 70% or more (i.e. 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or more) identity to SEQ ID NO: 28, in particular wherein the CDR sequences as defined above are maintained. In some embodiments, the antibody, or the antigen-binding fragment thereof, comprises a heavy chain comprising an amino acid sequence as set forth in SEQ ID NO: 25 and a light chain comprising the amino acid sequence as set forth in SEQ ID NO: 28.

More preferably, the antibody of the invention, or the antigen-binding fragment thereof, may comprise a heavy chain comprising an amino acid sequence having 70% or more (i.e. 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or more) identity to SEQ ID NO: 25 and a light chain comprising the amino acid sequence having 70% or more (i.e. 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or more) identity to SEQ ID NO: 29 or 30, in particular wherein the CDR sequences as defined above are maintained. In some embodiments, the antibody, or the antigen-binding fragment thereof, comprises a heavy chain comprising an amino acid sequence as set forth in SEQ ID NO: 25 and a light chain comprising the amino acid sequence as set forth in SEQ ID NO: 29 or 30.

As outlined above, an antibody according to the present invention may comprise a (complete) Fc region derived from human IgG4. In some embodiments, the antibody according to the present invention comprises, in particular in addition to a (complete) Fc region derived from human IgG4 also all other parts of the constant regions of IgG, such as all other parts of the constant regions of (human) IgG4.

In some embodiments, the antibody according to the present invention comprises a (complete) Fc moiety/Fc region, wherein the interaction/binding with FcR is not compromised.

In general, binding of the antibody to an Fc receptor may be assessed by various methods known to the skilled person, such as ELISA (Hessell AJ, Hangartner L, Hunter M, Havenith CEG, Beurskens FJ, Bakker JM, Lanigan CMS, Landucci G, Forthal DN, Parren PWHI, et al.: Fc receptor but not complement binding is important in antibody protection against HIV. Nature 2007, 449:101-104; Grevys A, Bern M, Foss S, Bratlie DB, Moen A, Gunnarsen KS, Aase A, Michaelsen TE, Sandlie I, Andersen JT: Fc Engineering of Human IgG1 for Altered Binding to the Neonatal Fc Receptor Affects Fc Effector Functions. 2015, 194:5497-5508) or flow-cytometry (Perez LG, Costa MR, Todd CA, Haynes BF, Montefiori DC: Utilization of immunoglobulin G Fc receptors by human immunodeficiency virus type 1: a specific role for antibodies against the membrane-proximal external region of gp41. J Virol 2009, 83:7397-7410; Piccoli L, Campo I, Fregni CS, Rodriguez BMF, Minola A, Sallusto F, Luisetti M, Corti D, Lanzavecchia A: Neutralization and clearance of GM-CSF by autoantibodies in pulmonary alveolar proteinosis. Nat Commun 2015, 6:1-9).

The antibody according to the present invention may be glycosylated. N-linked glycans attached to the CH2 domain of a heavy chain, for instance, can influence C1q and FcR binding, with glycosylated antibodies having lower affinity for these receptors. Accordingly, the CH2 domain of the Fc moiety of the antibody according to the present invention may comprise one or more mutations, in which a glycosylated residue is substituted by a non-glycosylated residue. For example, the antibody's glycans do not lead to a human immunogenic response after administration.

Furthermore, the antibody according to the present invention can be modified by introducing (random) amino acid mutations into particular region of the CH2 or CH3 domain of the heavy chain in order to alter their binding affinity for FcR and/or their serum half-life in comparison to unmodified antibodies. Examples of such modifications include, but are not limited to, substitutions of at least one amino acid from the heavy chain constant region selected from the group consisting of amino acid residues 250, 314, and 428. Further examples of such Fc modifications are described in Saxena A, Wu D. Advances in Therapeutic Fc Engineering - Modulation of IgG-Associated Effector Functions and Serum Half-life. Front Immunol. 2016;7:580, which is incorporated herein by reference. In some embodiments, the antibody may comprise the "YTE" mutations (M252Y/S254T/T256E; EU numbering). In some embodiments, the antibody may comprise the mutations M428L and/or N434S in the heavy chain constant region (EU numbering).

Antibodies of the invention also include hybrid antibody molecules that comprise the six CDRs from an antibody of the invention as defined above and one or more CDRs from another antibody to an antigen. For example, the antibody may be bispecific.

Variant antibodies are also included within the scope of the invention. Thus, variants of the sequences recited in the application are also included within the scope of the invention. Such variants include natural variants generated by somatic mutation *in vivo* during the immune response or *in vitro* upon culture of immortalized B cell clones. Alternatively, variants may arise due to the degeneracy of the genetic code or may be produced due to errors in transcription or translation.

Antibodies of the invention may be provided in purified form. Typically, the antibody will be present in a composition that is substantially free of other polypeptides *e.g.,* where less than 90% (by weight), usually less than 60% and more usually less than 50% of the composition is made up of other polypeptides.

### Nucleic Acids

In another aspect, the invention also provides a nucleic acid molecule or a combination of nucleic acid molecules comprising a polynucleotide encoding the antibody according to the present invention, or an antigen-binding fragment thereof, as described above.

The one or more polynucleoticle(s) may encode an antibody, in particular the VH and VL sequences thereof or the six CDRs thereof. An antibody may comprise separate heavy and light chains. In such a case, (i) the heavy chain (or heavy chain elements, such as HCDR1, HCDR2 and HCDR3, or VH) and (ii) the light chain (or light chain elements, such as LCDR1, LCDR2 and LCDR3, or VL) may be encoded by the same or distinct nucleic acid molecule(s). In other words, nucleic acids may encode the light chain and/or the heavy chain of an antibody. For example, the light chain and the heavy chain of the antibody may be encoded by the same nucleic acid molecule (e.g., in bicistronic manner). Alternatively, the light chain and the heavy chain of the antibody may be encoded by distinct nucleic acid molecules.

Examples of nucleic acid molecules and/or polynucleotides include, e.g., a recombinant polynucleotide, a vector, an oligonucleotide, an RNA molecule such as an rRNA, an mRNA, an miRNA, an siRNA, or a tRNA, or a DNA molecule such as a cDNA.

Due to the redundancy of the genetic code, the present invention also comprises sequence variants of nucleic acid sequences, which encode the same amino acid sequences. The polynucleotide encoding the antibody (or the complete nucleic acid molecule) may be optimized for expression of the antibody. For example, codon optimization of the nucleotide sequence may be used to improve the efficiency of translation in expression systems for the production of the antibody. Moreover, the nucleic acid molecule may comprise heterologous elements (i.e., elements, which in nature do not occur on the same nucleic acid molecule as the coding sequence for the (heavy or light chain of) an antibody. For example, a nucleic acid molecule may comprise a heterologous promotor, a heterologous enhancer, a heterologous UTR (e.g., for optimal translation/expression), a heterologous Poly-A-tail, and the like.

A nucleic acid molecule is a molecule comprising nucleic acid components. The term nucleic acid molecule usually refers to DNA or RNA molecules. It may be used synonymous with the term "polynucleotide", i.e. the nucleic acid molecule may consist of a polynucleotide encoding the antibody. Alternatively, the nucleic acid molecule may also comprise further elements in addition to the polynucleotide encoding the antibody. Typically, a nucleic acid molecule is a polymer comprising or consisting of nucleotide monomers which are covalently linked to each other by phosphodiester-bonds of a sugar/phosphate-backbone. The term "nucleic acid molecule" also encompasses modified nucleic acid molecules, such as base-modified, sugar-modified or backbone-modified etc. DNA or RNA molecules.

In general, the nucleic acid molecule may be manipulated to insert, delete or alter certain nucleic acid sequences. Changes from such manipulation include, but are not limited to, changes to introduce restriction sites, to amend codon usage, to add or optimize transcription and/or translation regulatory sequences, etc. It is also possible to change the nucleic acid to alter the encoded amino acids. For example, it may be useful to introduce one or more (*e.g.*, 1, 2, 3, 4, 5, 6, 7, 8, 9, 10, etc.) amino acid substitutions, deletions and/or insertions into the antibody's amino acid sequence. Such point mutations can modify effector functions, antigen-binding affinity, post-translational modifications, immunogenicity, etc., can introduce amino acids for the attachment of covalent groups (e.g., labels) or can introduce tags (e.g., for purification purposes). Alternatively, a mutation in a nucleic acid sequence may be "silent", i.e. not reflected in the amino acid sequence due to the redundancy of the genetic code. In general, mutations can be introduced in specific sites or can be introduced at random, followed by selection (e.g., molecular evolution). For instance, one or more nucleic acids encoding any of the light or heavy chains of an (exemplary) antibody can be randomly or directionally mutated to introduce different properties in the encoded amino acids. Such changes can be the result of an iterative process wherein initial changes are retained and new changes at other nucleotide positions are introduced. Further, changes achieved in independent steps may be combined.

In some embodiments, the polynucleotide encoding the antibody, or an antigen-binding fragment thereof, (or the (complete) nucleic acid molecule) may be codon-optimized. The skilled artisan is aware of various tools for codon optimization, such as those described in: Ju Xin Chin, Bevan Kai-Sheng Chung, Dong-Yup Lee, Codon Optimization OnLine (COOL): a web-based multi-objective optimization platform for synthetic gene design, Bioinformatics, Volume 30, Issue 15, 1 August 2014, Pages 2210-2212; or in: Grote A, Hiller K, Scheer M, Munch R, Nortemann B, Hempel DC, Jahn D, JCat: a novel tool to adapt codon usage of a target gene to its potential expression host. Nucleic Acids Res. 2005 Jul 1;33(Web Server issue):W526-31; or, for example, Genscript's OptimumGene™ algorithm (as described in US 2011/0081708 A1).

The present invention also provides a combination of a first and a second nucleic acid molecule, wherein the first nucleic acid molecule comprises a polynucleotide encoding the heavy chain of the antibody, or an antigen-binding fragment thereof, of the present invention; and the second nucleic acid molecule comprises a polynucleotide encoding the corresponding light chain of the same antibody, or the same antigen-binding fragment thereof. The above description regarding the (general) features of the nucleic acid molecule of the invention applies accordingly to the first and second nucleic acid molecule of the combination. Accordingly, one or both of the polynucleotides encoding the heavy and/or light chain(s) of the antibody, or an antigen-binding fragment thereof, is/are codon-optimized.

### Vector

Further included within the scope of the invention are vectors, for example, expression vectors, comprising a nucleic acid molecule according to the present invention. Usually, a vector comprises a nucleic acid molecule as described above.

The present invention also provides a combination of a first and a second vector, wherein the first vector comprises a first nucleic acid molecule as described above (for the combination of nucleic acid molecules) and the second vector comprises a second nucleic acid molecule as described above (for the combination of nucleic acid molecules).

A vector is in particular a recombinant nucleic acid molecule, i.e. a nucleic acid molecule which does not occur in nature. Accordingly, the vector may comprise heterologous elements (i.e., sequence elements of different origin in nature). For example, the vector may comprise a multi cloning site, a heterologous promotor, a heterologous enhancer, a heterologous selection marker (to identify cells comprising said vector in comparison to cells not comprising said vector) and the like. A vector in the context of the present invention is suitable for incorporating or harboring a desired nucleic acid sequence. Such vectors may be storage vectors, expression vectors, cloning vectors, transfer vectors etc. A storage vector is a vector which allows the convenient storage of a nucleic acid molecule. Thus, the vector may comprise a sequence corresponding, e.g., to a (heavy and/or light chain of a) desired antibody according to the present invention. An expression vector may be used for production of expression products such as RNA, e.g. mRNA, or peptides, polypeptides or proteins. For example, an expression vector may comprise sequences needed for transcription of a sequence stretch of the vector, such as a (heterologous) promoter sequence. A cloning vector is typically a vector that contains a cloning site, which may be used to incorporate nucleic acid sequences into the vector. A cloning vector may be, e.g., a plasmid vector or a bacteriophage vector. A transfer vector may be a vector which is suitable for transferring nucleic acid molecules into cells or organisms, for example, viral vectors. A vector in the context of the present invention may be, e.g., an RNA vector or a DNA vector. For example, a vector in the sense of the present application comprises a cloning site, a selection marker, such as an antibiotic resistance factor, and a sequence suitable for multiplication of the vector, such as an origin of replication. A vector in the context of the present application may be a plasmid vector.

### Cells

In a further aspect, the present invention also provides cell expressing the antibody according to the present invention; and/or comprising the vector (or the combination of vectors) according the present invention.

Examples of such cells include but are not limited to, eukaryotic cells, e.g., yeast cells, animal cells or plant cells. Other examples of such cells include but are not limited, to prokaryotic cells, e.g. *E. coli.* In some embodiments, the cells are mammalian cells, such as a mammalian cell line. Examples include human cells, CHO cells, HEK293T cells, PER.C6 cells, NS0 cells, human liver cells, myeloma cells or hybridoma cells.

The cell may be transfected with a vector according to the present invention, for example with an expression vector. The term "transfection" refers to the introduction of nucleic acid molecules, such as DNA or RNA (e.g. mRNA) molecules, into cells, e.g. into eukaryotic or prokaryotic cells. In the context of the present invention, the term "transfection" encompasses any method known to the skilled person for introducing nucleic acid molecules into cells, such as into mammalian cells. Such methods encompass, for example, electroporation, lipofection, e.g. based on cationic lipids and/or liposomes, calcium phosphate precipitation, nanoparticle based transfection, virus based transfection, or transfection based on cationic polymers, such as DEAE-dextran or polyethylenimine etc. In some embodiments, the introduction is non-viral.

Moreover, the cells of the present invention may be transfected stably or transiently with the vector according to the present invention, e.g. for expressing the antibody according to the present invention. In some embodiments, the cells are stably transfected with the vector according to the present invention encoding the antibody according to the present invention. In other embodiments, the cells are transiently transfected with the vector according to the present invention encoding the antibody according to the present invention.

Accordingly, the present invention also provides a recombinant host cell, which heterologously expresses the antibody of the invention or the antigen-binding fragment thereof. For example, the cell may be of another species than the antibody (e.g., CHO cells expressing human antibodies). In some embodiments, the cell type of the cell does not express (such) antibodies in nature. Moreover, the host cell may impart a post-translational modification (PTM; e.g., glycosylation) on the antibody that is not present in their native state. Such a PTM may result in a functional difference (e.g., reduced immunogenicity). Accordingly, the antibody of the invention, or the antigen-binding fragment thereof, may have a post-translational modification, which is distinct from the naturally produced antibody (e.g., an antibody of an immune response in a human).

### Production of Antibodies

Antibodies according to the invention can be made by any method known in the art. For example, the general methodology for making monoclonal antibodies using hybridoma technology is well known (Kohler, G. and Milstein, C,. 1975; Kozbar et al. 1983).

In some embodiments, the method as described in WO 2004/076677, which is incorporated herein by reference, is used. In this method B cells producing the antibody of the invention are transformed with EBV and a polyclonal B cell activator. Additional stimulants of cellular growth and differentiation may optionally be added during the transformation step to further enhance the efficiency. These stimulants may be cytokines such as IL-2 and IL-15. In one aspect, IL-2 is added during the immortalization step to further improve the efficiency of immortalization, but its use is not essential. The immortalized B cells produced using these methods can then be cultured using methods known in the art and antibodies isolated therefrom.

Another exemplified method is described in WO 2010/046775. In this method plasma cells are cultured in limited numbers, or as single plasma cells in microwell culture plates. Antibodies can be isolated from the plasma cell cultures. Further, from the plasma cell cultures, RNA can be extracted and PCR can be performed using methods known in the art. The VH and VL regions of the antibodies can be amplified by RT-PCR (reverse transcriptase PCR), sequenced and cloned into an expression vector that is then transfected into HEK293T cells or other host cells. The cloning of nucleic acid in expression vectors, the transfection of host cells, the culture of the transfected host cells and the isolation of the produced antibody can be done using any methods known to one of skill in the art.

The antibodies may be further purified, if desired, using filtration, centrifugation and various chromatographic methods such as HPLC or affinity chromatography. Techniques for purification of antibodies, e.g., monoclonal antibodies, including techniques for producing pharmaceutical-grade antibodies, are well known in the art.

Standard techniques of molecular biology may be used to prepare DNA sequences encoding the antibodies of the present invention. Desired DNA sequences may be synthesized completely or in part using oligonucleotide synthesis techniques. Site-directed mutagenesis and polymerase chain reaction (PCR) techniques may be used as appropriate.

Any suitable host cell/vector system may be used for expression of the DNA sequences encoding the antibody molecules of the present invention. Eukaryotic, *e.g*., mammalian, host cell expression systems may be used for production of antibody molecules, such as complete antibody molecules. Suitable mammalian host cells include, but are not limited to, CHO, HEK293T, PER.C6, NS0, myeloma or hybridoma cells. Also, prokaryotic, e.g. bacterial host cell expression systems may be used for the production of antibody molecules, such as complete antibody molecules. Suitable bacterial host cells include, but are not limited to, *E*. *coli* cells.

The present invention also provides a process for the production of an antibody molecule according to the present invention comprising culturing a (heterologous) host cell comprising a vector encoding a nucleic acid of the present invention under conditions suitable for expression of protein from DNA encoding the antibody molecule of the present invention, and isolating the antibody molecule.

For production of the antibody comprising both heavy and light chains, a cell line may be transfected with two vectors, a first vector encoding a light chain polypeptide and a second vector encoding a heavy chain polypeptide. Alternatively, a single vector may be used, the vector including sequences encoding light chain and heavy chain polypeptides.

Antibodies according to the invention may be produced by (i) expressing a nucleic acid sequence according to the invention in a host cell, e.g. by use of a vector according to the present invention, and (ii) isolating the expressed antibody product. Additionally, the method may include (iii) purifying the isolated antibody. Transformed B cells and cultured plasma cells may be screened for those producing antibodies of the desired specificity or function.

The screening step may be carried out by any immunoassay, *e.g.*, ELISA, by staining of tissues or cells (including transfected cells), by neutralization assay or by one of a number of other methods known in the art for identifying desired specificity or function. The assay may select on the basis of simple recognition of one or more antigens, or may select on the additional basis of a desired function *e.g.*, to select neutralizing antibodies rather than just antigen-binding antibodies, to select antibodies that can change characteristics of targeted cells, such as their signaling cascades, their shape, their growth rate, their capability of influencing other cells, their response to the influence by other cells or by other reagents or by a change in conditions, their differentiation status, etc.

Individual transformed B cell clones may then be produced from the positive transformed B cell culture. The cloning step for separating individual clones from the mixture of positive cells may be carried out using limiting dilution, micromanipulation, single cell deposition by cell sorting or another method known in the art.

Nucleic acid from the cultured plasma cells can be isolated, cloned and expressed in HEK293T cells or other known host cells using methods known in the art.

The immortalized B cell clones or the transfected host-cells of the invention can be used in various ways e.g., as a source of monoclonal antibodies, as a source of nucleic acid (DNA or mRNA) encoding a monoclonal antibody of interest, for research, etc.

The invention also provides a composition comprising immortalized B memory cells or transfected host cells that produce antibodies according to the present invention.

The immortalized B cell clone or the cultured plasma cells of the invention may also be used as a source of nucleic acid for the cloning of antibody genes for subsequent recombinant expression. Expression from recombinant sources may be more common for pharmaceutical purposes than expression from B cells or hybridomas e.g., for reasons of stability, reproducibility, culture ease, etc.

Thus the invention also provides a method for preparing a recombinant cell, comprising the steps of: (i) obtaining one or more nucleic acids (*e.g.*, heavy and/or light chain mRNAs) from the B cell clone or the cultured plasma cells that encodes the antibody of interest; (ii) inserting the nucleic acid into an expression vector and (iii) transfecting the vector into a (heterologous) host cell in order to permit expression of the antibody of interest in that host cell.

Similarly, the invention also provides a method for preparing a recombinant cell, comprising the steps of: (i) sequencing nucleic acid(s) from the B cell clone or the cultured plasma cells that encodes the antibody of interest; and (ii) using the sequence information from step (i) to prepare nucleic acid(s) for insertion into a host cell in order to permit expression of the antibody of interest in that host cell. The nucleic acid may, but need not, be manipulated between steps (i) and (ii) to introduce restriction sites, to change codon usage, and/or to optimize transcription and/or translation regulatory sequences.

Furthermore, the invention also provides a method of preparing a transfected host cell, comprising the step of transfecting a host cell with one or more nucleic acids that encode an antibody of interest, wherein the nucleic acids are nucleic acids that were derived from an immortalized B cell clone or a cultured plasma cell of the invention. Thus the procedures for first preparing the nucleic acid(s) and then using it to transfect a host cell can be performed at different times by different people in different places (e.g., in different countries).

These recombinant cells of the invention can then be used for expression and culture purposes. They are particularly useful for expression of antibodies for large-scale pharmaceutical production. They can also be used as the active ingredient of a pharmaceutical composition. Any suitable culture technique can be used, including but not limited to static culture, roller bottle culture, ascites fluid, hollow-fiber type bioreactor cartridge, modular minifermenter, stirred tank, microcarrier culture, ceramic core perfusion, etc.

Methods for obtaining and sequencing immunoglobulin genes from B cells or plasma cells are well known in the art (e.g., see Chapter 4 of Kuby Immunology, 4th edition, 2000).

The transfected host cell may be a eukaryotic cell, including yeast and animal cells, particularly mammalian cells (e.g., CHO cells, NS0 cells, human cells such as PER.C6 or HKB-11 cells, myeloma cells, or a human liver cell), as well as plant cells. In some embodiments, the transfected host cell is a mammalian cell, such as a human cell. In some embodiments, expression hosts can glycosylate the antibody of the invention, particularly with carbohydrate structures that are not themselves immunogenic in humans. In some embodiments the transfected host cell may be able to grow in serum-free media. In further embodiments the transfected host cell may be able to grow in culture without the presence of animal-derived products. The transfected host cell may also be cultured to give a cell line.

The invention also provides a method for preparing one or more nucleic acid molecules (e.g., heavy and light chain genes) that encode an antibody of interest, comprising the steps of:
(i) preparing an immortalized B cell clone or culturing plasma cells according to the invention; (ii) obtaining from the B cell clone or the cultured plasma cells nucleic acid that encodes the antibody of interest. Further, the invention provides a method for obtaining a nucleic acid sequence that encodes an antibody of interest, comprising the steps of: (i) preparing an immortalized B cell clone or culturing plasma cells according to the invention;
(ii) sequencing nucleic acid from the B cell clone or the cultured plasma cells that encodes the antibody of interest.

The invention further provides a method of preparing nucleic acid molecule(s) that encode an antibody of interest, comprising the step of obtaining the nucleic acid that was obtained from a transformed B cell clone or cultured plasma cells of the invention. Thus the procedures for first obtaining the B cell clone or the cultured plasma cell, and then obtaining nucleic acid(s) from the B cell clone or the cultured plasma cells can be performed at different times by different people in different places (e.g., in different countries).

The invention also comprises a method for preparing an antibody (e.g., for pharmaceutical use) according to the present invention, comprising the steps of: (i) obtaining and/or sequencing one or more nucleic acids (*e.g*., heavy and light chain genes) from the selected B cell clone or the cultured plasma cells expressing the antibody of interest; (ii) inserting the nucleic acid(s) into or using the nucleic acid(s) sequence(s) to prepare an expression vector; (iii) transfecting a host cell that can express the antibody of interest; (iv) culturing or sub-culturing the transfected host cells under conditions where the antibody of interest is expressed; and, optionally, (v) purifying the antibody of interest.

The invention also provides a method of preparing the antibody of interest comprising the steps of: culturing or sub-culturing a transfected host cell population, e.g. a stably transfected host cell population, under conditions where the antibody of interest is expressed and, optionally, purifying the antibody of interest, wherein said transfected host cell population has been prepared by (i) providing nucleic acid(s) encoding a selected antibody of interest that is produced by a B cell clone or cultured plasma cells prepared as described above, (ii) inserting the nucleic acid(s) into an expression vector, (iii) transfecting the vector in a host cell that can express the antibody of interest, and (iv) culturing or sub-culturing the transfected host cell comprising the inserted nucleic acids to produce the antibody of interest. Thus the procedures for first preparing the recombinant host cell and then culturing it to express antibody can be performed at very different times by different people in different places (e.g., in different countries).

### Pharmaceutical Composition

The present invention also provides a pharmaceutical composition comprising one or more of:
(i) the antibody according to the present invention, or an antigen-binding fragment thereof;
(ii) the nucleic acid or a combination of nucleic acids according to the present invention;
(iii) the vector or a combination of vectors according to the present invention; and/or
(iv) the cell expressing the antibody according to the present invention or comprising the nucleic acid or vector according to the present invention
and, optionally, a pharmaceutically acceptable diluent or carrier.

In other words, the present invention also provides a pharmaceutical composition comprising the antibody according to the present invention, the nucleic acid according to the present invention, the vector according to the present invention and/or the cell according to the present invention.

The pharmaceutical composition may optionally also contain a pharmaceutically acceptable carrier, diluent and/or excipient. Although the carrier or excipient may facilitate administration, it should not itself induce the production of antibodies harmful to the individual receiving the composition. Nor should it be toxic. Suitable carriers may be large, slowly metabolized macromolecules such as proteins, polypeptides, liposomes, polysaccharides, polylactic acids, polyglycolic acids, polymeric amino acids, amino acid copolymers and inactive virus particles. In particular, the pharmaceutically acceptable carrier, diluent and/or excipient in the pharmaceutical composition according to the present invention is not an active component in respect to a disease to be treated with the antibody, such as diseases treated by natalizumab, such as multiple sclerosis or Crohn's disease. Pharmaceutically acceptable salts can be used, for example mineral acid salts, such as hydrochlorides, hydrobromides, phosphates and sulphates, or salts of organic acids, such as acetates, propionates, malonates and benzoates.

Pharmaceutically acceptable carriers in a pharmaceutical composition may additionally contain liquids such as water, saline, glycerol and ethanol. Additionally, auxiliary substances, such as wetting or emulsifying agents or pH buffering substances, may be present *in* such compositions. Such carriers enable the pharmaceutical compositions to be formulated as tablets, pills, dragees, capsules, liquids, gels, syrups, slurries and suspensions, for ingestion by the subject.

Pharmaceutical compositions of the invention may be prepared in various forms. For example, the compositions may be prepared as injectables, either as liquid solutions or suspensions. Solid forms suitable for solution in, or suspension in, liquid vehicles prior to injection can also be prepared (e.g., a lyophilized composition, similar to Synagis™ and Herceptin®, for reconstitution with sterile water containing a preservative). The composition may be prepared for topical administration e.g., as an ointment, cream or powder. The composition may be prepared for oral administration e.g., as a tablet or capsule, as a spray, or as a syrup (optionally flavored). The composition may be prepared for pulmonary administration e.g., as an inhaler, using a fine powder or a spray. The composition may be prepared as a suppository or pessary. The composition may be prepared for nasal, aural or ocular administration e.g., as drops. The composition may be in kit form, designed such that a combined composition is reconstituted just prior to administration to a subject. For example, a lyophilized antibody may be provided in kit form with sterile water or a sterile buffer.

In some embodiments, the (only) active ingredient in the composition is the antibody according to the present invention. As such, it may be susceptible to degradation in the gastrointestinal tract. Thus, if the composition is to be administered by a route using the gastrointestinal tract, the composition may contain agents which protect the antibody from degradation but which release the antibody once it has been absorbed from the gastrointestinal tract.

A thorough discussion of pharmaceutically acceptable carriers is available in Gennaro (2000) Remington: The Science and Practice of Pharmacy, 20th edition, ISBN: 0683306472.

Pharmaceutical compositions of the invention generally have a pH between 5.5 and 8.5, in some embodiments this may be between 6 and 8, for example about 7. The pH may be maintained by the use of a buffer. The composition may be sterile and/or pyrogen free. The composition may be isotonic with respect to humans. In some embodiments pharmaceutical compositions of the invention are supplied in hermetically-sealed containers.

Within the scope of the invention are compositions present in several forms of administration; the forms include, but are not limited to, those forms suitable for parenteral administration, e.g., by injection or infusion, for example by bolus injection or continuous infusion. Where the product is for injection or infusion, it may take the form of a suspension, solution or emulsion in an oily or aqueous vehicle and it may contain formulatory agents, such as suspending, preservative, stabilizing and/or dispersing agents. Alternatively, the antibody may be in dry form, for reconstitution before use with an appropriate sterile liquid.

A vehicle is typically understood to be a material that is suitable for storing, transporting, and/or administering a compound, such as a pharmaceutically active compound, in particular the antibodies according to the present invention. For example, the vehicle may be a physiologically acceptable liquid, which is suitable for storing, transporting, and/or administering a pharmaceutically active compound, in particular the antibodies according to the present invention. Once formulated, the compositions of the invention can be administered directly to the subject. In some embodiments the compositions are adapted for administration to mammalian, e.g., human subjects.

The pharmaceutical compositions of this invention may be administered by any number of routes including, but not limited to, oral, intravenous, intramuscular, intra-arterial, intramedullary, intraperitoneal, intrathecal, intraventricular, transdermal, transcutaneous, topical, subcutaneous, intranasal, enteral, sublingual, intravaginal or rectal routes. Hyposprays may also be used to administer the pharmaceutical compositions of the invention. Optionally, the pharmaceutical composition may be prepared for oral administration, e.g. as tablets, capsules and the like, for topical administration, or as injectable, e.g. as liquid solutions or suspensions. In some embodiments, the pharmaceutical composition is an injectable. Solid forms suitable for solution in, or suspension in, liquid vehicles prior to injection are also encompassed, for example the pharmaceutical composition may be in lyophilized form.

The antibody may be administered by injection or infusion, for example by intravenous infusion. For injection/infusion, e.g. intravenous, cutaneous or subcutaneous injection/infusion, or injection at the site of affliction, the active ingredient may be in the form of a parenterally acceptable aqueous solution which is pyrogen-free and has suitable pH, isotonicity and stability. Those of relevant skill in the art are well able to prepare suitable solutions using, for example, isotonic vehicles such as Sodium Chloride Injection, Ringer's Injection, Lactated Ringer's Injection. Preservatives, stabilizers, buffers, antioxidants and/or other additives may be included, as required. Whether it is an antibody, a peptide, a nucleic acid molecule, or another pharmaceutically useful compound according to the present invention that is to be given to an individual, administration is usually in a "prophylactically effective amount" or a "therapeutically effective amount" (as the case may be), this being sufficient to show benefit to the individual. The actual amount administered, and rate and time-course of administration, will depend on the nature and severity of what is being treated. For injection, the pharmaceutical composition according to the present invention may be provided for example in a pre-filled syringe.

The inventive pharmaceutical composition as defined above may also be administered orally in any orally acceptable dosage form including, but not limited to, capsules, tablets, aqueous suspensions or solutions. In the case of tablets for oral use, carriers commonly used include lactose and corn starch. Lubricating agents, such as magnesium stearate, are also typically added. For oral administration in a capsule form, useful diluents include lactose and dried cornstarch. When aqueous suspensions are required for oral use, the active ingredient, i.e. the inventive transporter cargo conjugate molecule as defined above, is combined with emulsifying and suspending agents. If desired, certain sweetening, flavoring or coloring agents may also be added.

The inventive pharmaceutical composition may also be administered topically, especially when the target of treatment includes areas or organs readily accessible by topical application, e.g. including accessible epithelial tissue. Suitable topical formulations are readily prepared for each of these areas or organs. For topical applications, the inventive pharmaceutical composition may be formulated in a suitable ointment, containing the inventive pharmaceutical composition, particularly its components as defined above, suspended or dissolved in one or more carriers. Carriers for topical administration include, but are not limited to, mineral oil, liquid petrolatum, white petrolatum, propylene glycol, polyoxyethylene, polyoxypropylene compound, emulsifying wax and water. Alternatively, the inventive pharmaceutical composition can be formulated in a suitable lotion or cream. In the context of the present invention, suitable carriers include, but are not limited to, mineral oil, sorbitan monostearate, polysorbate 60, cetyl esters wax, cetearyl alcohol, 2-octyldodecanol, benzyl alcohol and water.

Dosage treatment may be a single dose schedule or a multiple dose schedule. In particular, the pharmaceutical composition may be provided as single-dose product. In some embodiments, the amount of the antibody in the pharmaceutical composition - in particular if provided as single-dose product - does not exceed 500 mg, for example it does not exceed 400 mg or 300 mg.

For a single dose, e.g. a daily, weekly or monthly dose, the amount of the antibody in the pharmaceutical composition according to the present invention, may not exceed 1 g or 500 mg. In some embodiments, for a single dose, the amount of the antibody in the pharmaceutical composition according to the present invention, may not exceed 400 mg, or 300 mg. For example, the antibody, or the antigen binding fragment thereof, may be administered at a single dose of 300 mg every 28 days.

Pharmaceutical compositions typically include an "effective" amount of one or more antibodies of the invention, i.e. an amount that is sufficient to treat, ameliorate, attenuate, reduce or prevent a desired disease or condition, or to exhibit a detectable therapeutic effect. Therapeutic effects also include reduction or attenuation in pathogenic potency or physical symptoms. The precise effective amount for any particular subject will depend upon their size, weight, and health, the nature and extent of the condition, and the therapeutics or combination of therapeutics selected for administration. The effective amount for a given situation is determined by routine experimentation and is within the judgment of a clinician. For purposes of the present invention, an effective dose may generally be from about 0.005 to about 100 mg/kg, for example from about 0.0075 to about 50 mg/kg or from about 0.01 to about 10 mg/kg. In some embodiments, the effective dose will be from about 0.02 to about 5 mg/kg, of the antibody of the present invention (e.g. amount of the antibody in the pharmaceutical composition) in relation to the bodyweight (e.g., in kg) of the individual to which it is administered.

In some embodiments, a composition of the invention may include antibodies of the invention, wherein the antibodies may make up at least 50% by weight (*e.g.*, 60%, 70%, 75%, 80%, 85%, 90%, 95%, 96%, 97%, 98%, 99% or more) of the total protein in the composition. In the composition of the invention, the antibodies may be in purified form.

The present invention also provides a method of preparing a pharmaceutical composition comprising the steps of: (i) preparing an antibody of the invention; and (ii) admixing the purified antibody with one or more pharmaceutically-acceptable carriers.

In other embodiments, a method of preparing a pharmaceutical composition comprises the step of: admixing an antibody with one or more pharmaceutically-acceptable carriers, wherein the antibody is a monoclonal antibody that was obtained from a transformed B cell or a cultured plasma cell of the invention.

As an alternative to delivering antibodies or B cells for therapeutic purposes, it is possible to deliver nucleic acid (typically DNA) that encodes the monoclonal antibody of interest derived from the B cell or the cultured plasma cells to a subject, such that the nucleic acid can be expressed in the subject *in situ* to provide a desired therapeutic effect. Suitable gene therapy and nucleic acid delivery vectors are known in the art.

Pharmaceutical compositions may include an antimicrobial, particularly if packaged in a multiple dose format. They may comprise detergent e.g., a Tween (polysorbate), such as Tween 80. Detergents are generally present at low levels e.g., less than 0.01%. Compositions may also include sodium salts (e.g., sodium chloride) to give tonicity. For example, a concentration of 10±2mg/ml NaCl is typical.

Further, pharmaceutical compositions may comprise a sugar alcohol (e.g., mannitol) or a disaccharide (e.g., sucrose or trehalose) e.g., at around 15-30 mg/ml (e.g., 25 mg/ml), particularly if they are to be lyophilized or if they include material which has been reconstituted from lyophilized material. The pH of a composition for lyophilization may be adjusted to between 5 and 8, or between 5.5 and 7, or around 6.1 prior to lyophilization.

The compositions of the invention may also comprise one or more immunoregulatory agents. In some embodiments, one or more of the immunoregulatory agents include(s) an adjuvant. In other embodiments, the composition does not comprise an immunoregulatory agent.

### Medical Treatments and Uses

In a further aspect, the present invention provides the antibody according to the present invention, or an antigen-binding fragment thereof, the nucleic acid molecule (or the combination of nucleic acid molecules) according to the present invention, the vector (or the combination of vectors) according to the present invention, the cell according to the present invention or the pharmaceutical composition according to the present invention for use in medicine. In particular, the antibody according to the present invention, or an antigen-binding fragment thereof, the nucleic acid molecule (or the combination of nucleic acid molecules) according to the present invention, the vector (or the combination of vectors) according to the present invention, the cell according to the present invention or the pharmaceutical composition according to the present invention may be used in prophylaxis and/or treatment of multiple sclerosis or Crohn's disease; or in (ii) diagnosis of multiple sclerosis or Crohn's disease. Accordingly, the present invention also provides a method of reducing multiple sclerosis or Crohn's disease, or lowering the risk of multiple sclerosis or Crohn's disease, comprising: administering to a subject in need thereof, a therapeutically effective amount of the antibody, or an antigen-binding fragment thereof, according to the present invention, the nucleic acid molecule (or the combination of nucleic acid molecules) according to the present invention, the vector (or the combination of vectors) according to the present invention, the cell according to the present invention or the pharmaceutical composition according to the present invention. Moreover, the present invention also provides the use of the antibody according to the present invention, or an antigen-binding fragment thereof, the nucleic acid molecule (or the combination of nucleic acid molecules) according to the present invention, the vector (or the combination of vectors) according to the present invention, the cell according to the present invention or the pharmaceutical composition according to the present invention in the manufacture of a medicament for prophylaxis, treatment or attenuation of multiple sclerosis or Crohn's disease.

Prophylaxis of multiple sclerosis or Crohn's disease refers in particular to prophylactic settings, wherein the subject was not diagnosed with multiple sclerosis or Crohn's disease (either no diagnosis was performed or diagnosis results were negative) and/or the subject does not show symptoms of multiple sclerosis or Crohn's disease. In therapeutic settings, in contrast, the subject is typically diagnosed with multiple sclerosis or Crohn's disease and/or showing symptoms of multiple sclerosis or Crohn's disease. Of note, the terms "treatment" and "therapy"/"therapeutic" of multiple sclerosis or Crohn's disease include (complete) cure as well as attenuation/reduction of multiple sclerosis or Crohn's disease and/or related symptoms.

### BRIEF DESCRIPTION OF THE FIGURES

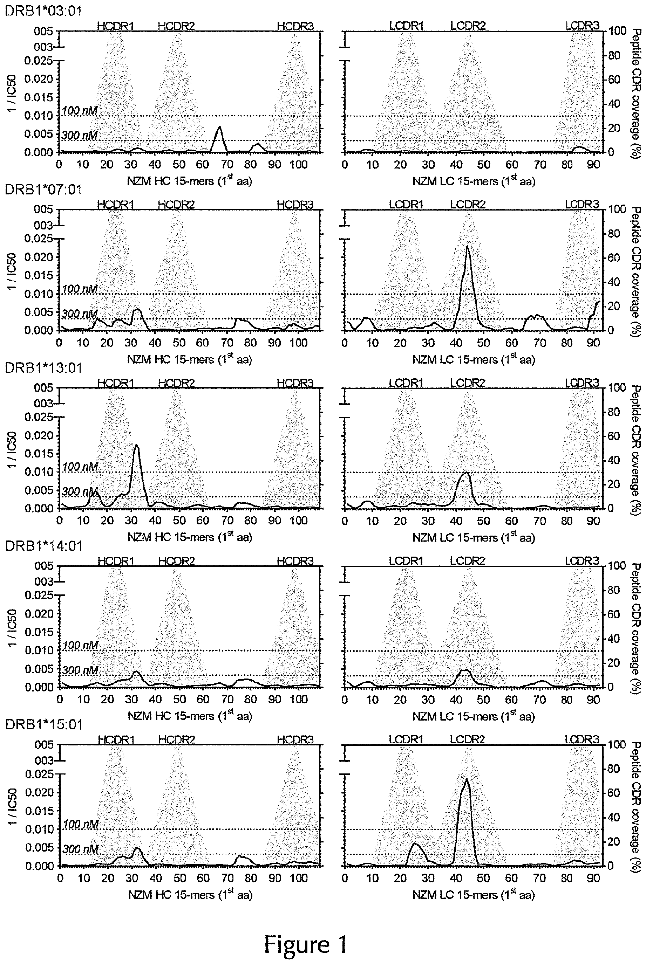

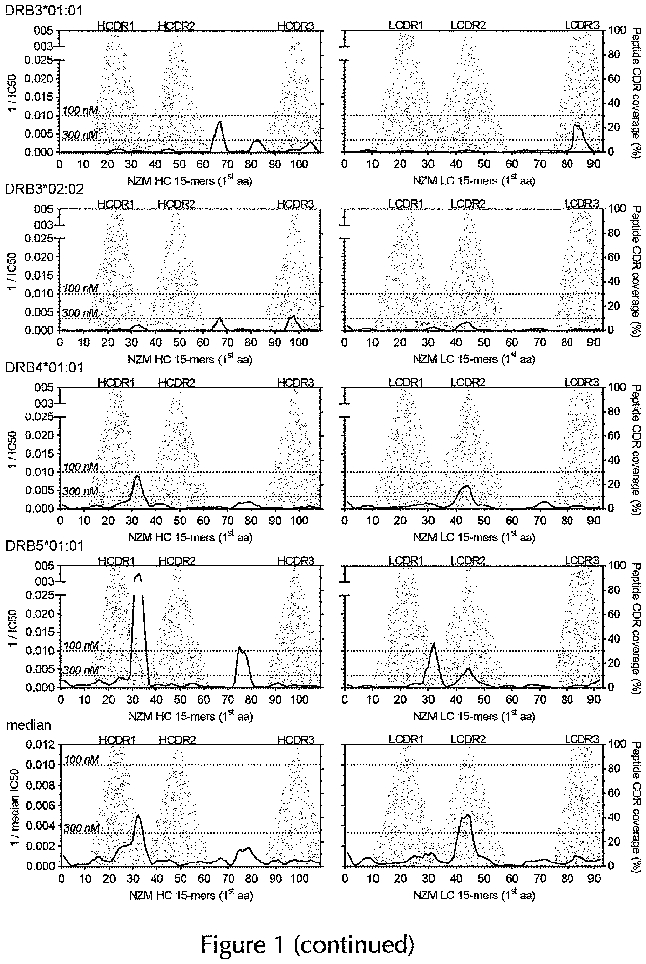

- Figure 1: shows the predicted binding affinities of all theoretical 15mer peptides derived from NZM heavy chain (HC) and light chain (LC) to a reference set of nine HLA-DRB1 and HLA-DRB3/4/5 alleles including DRB1^{∗}13/14 that were found to be associated to NZM-related allergic reactions. The affinities are shown as reciprocal IC50 (nM) values. The dotted lines define the thresholds of high-affinity binding set at 100 nM and low-affinity binding set at 300 nM.

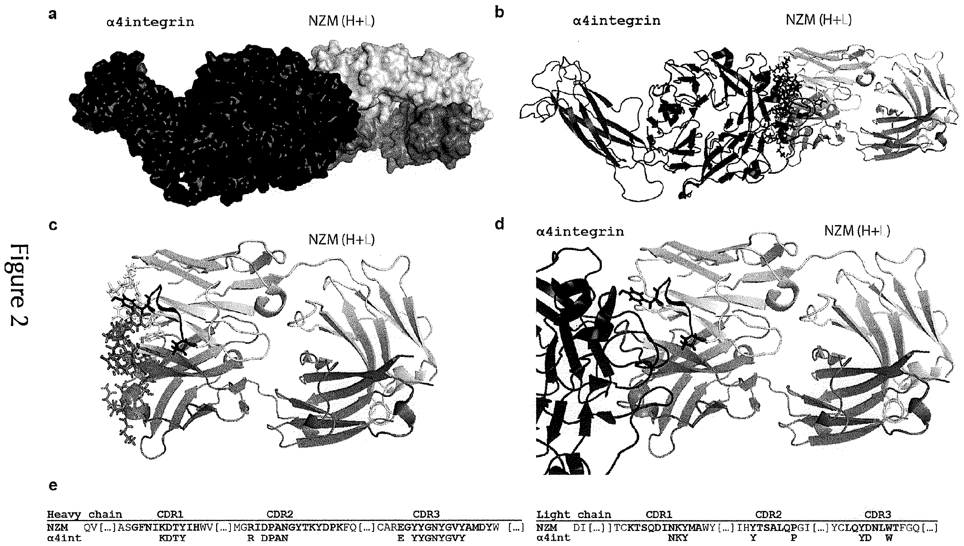

- Figure 2: shows a detailed visualization of the interacting interfaces of α4 integrin and NZM. a, The molecules are shown as ribbon diagrams with overlapping surfaces. b, The residues of the six CDRs of NZM that were positioned at less than 4.0 Å are shown as sticks. c, Close-up view of the LCDR2 loop of NZM. d, Close-up view of the LCDR2 of NZM showing that only two residues that are positioned at the far ends of the loop interact with α4-integrin. e, The alignment shows the NZM residues that are engaged by the interaction with α4-integrin.

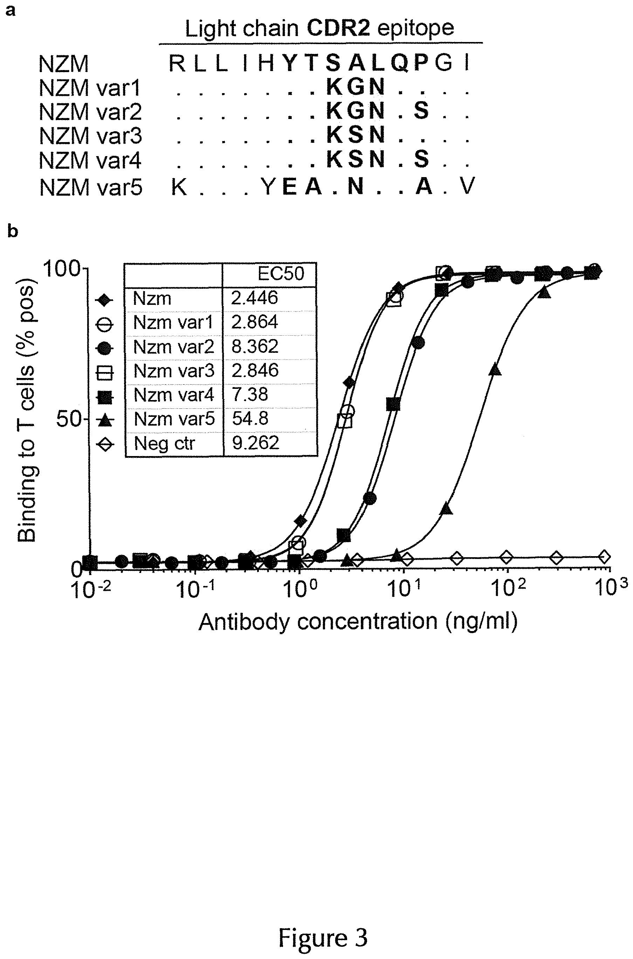

- Figure 3: shows the binding of the 5 NZM variants to α4-integrin expressed on T cells. a, The alignment shows the NZM LCDR2 and FR residues that are mutated in the five variants. b, Comparison of binding curves and EC50 values of the NZM and the 5 variants.

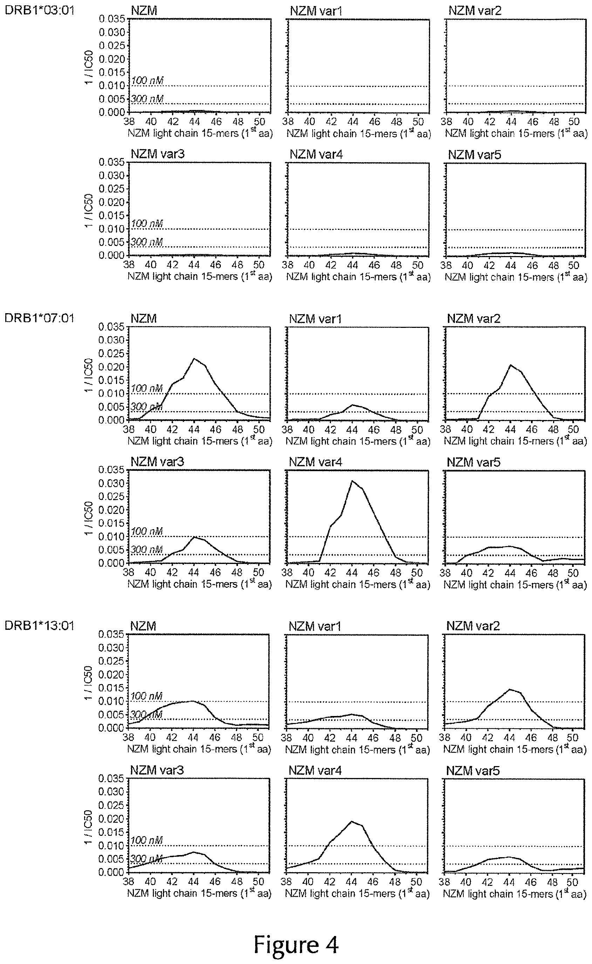

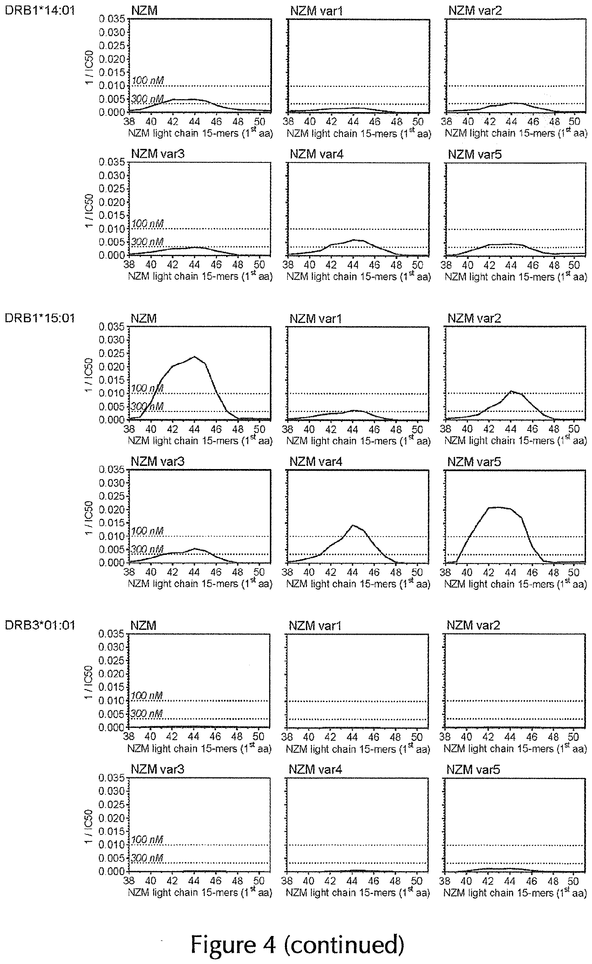

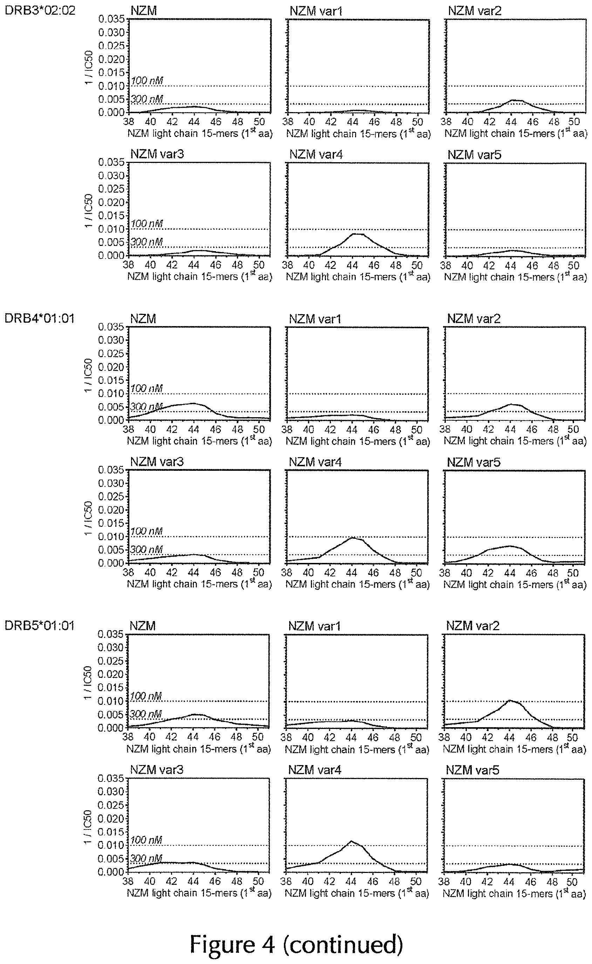

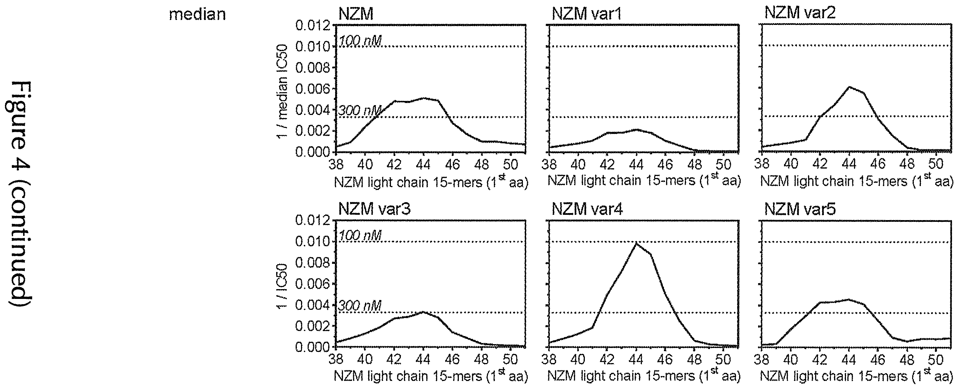

- Figure 4: shows the predicted binding affinities of all theoretical 15mer peptides derived from LCDR2 of NZM and the 5 variants to a reference set of nine HLA-DRB1 and HLA-DRB3/4/5 alleles including DRB1^{∗}13/14 that were found to be associated to NZM-related allergic reactions. The affinities are shown as reciprocal IC50 (nM) values. The dotted lines define the thresholds of high-affinity binding set at 100 nM and low-affinity binding set at 300 nM.

### EXAMPLES

In the following, particular examples illustrating various embodiments and aspects of the invention are presented. However, the present invention shall not to be limited in scope by the specific embodiments described herein. The following preparations and examples are given to enable those skilled in the art to more clearly understand and to practice the present invention. The present invention, however, is not limited in scope by the exemplified embodiments, which are intended as illustrations of single aspects of the invention only, and methods which are functionally equivalent are within the scope of the invention. Indeed, various modifications of the invention in addition to those described herein will become readily apparent to those skilled in the art from the foregoing description, accompanying figures and the examples below. All such modifications fall within the scope of the appended claims.

### Example 1: Identification of a single T cell epitope in the CDR2 of NZM light chain responsible for MHC binding

In view of the immunogenicity observed for natalizumab (NZM), NZM was investigated in order to identify the region(s) of NZM responsible for said immunogenicity.

All theoretical 15mer peptides spanning the heavy and light chain variable regions of NZM (NZM VH and VL) were investigated for their *in silico* binding affinity to major histocompatibility complex (MHC) class II molecules. To this end, NetMHCIIpan 3.2 server (Jensen KK, Andreatta M, Marcatili P, Buus S, Greenbaum JA, Yan Z, Sette A, Peters B, Nielsen M.: Improved methods for predicting peptide binding affinity to MHC class II molecules. Immunology. 2018 Jan 6. doi: 10.1111/imm.12889; URL: http://www.cbs.dtu.dk/services/NetMHCIIpan-3.2/) was used to predict the IC50 (nM) values of all theoretical NZM-derived peptides (15mers overlapping of 14) binding to a reference set of nine HLA-DRB1 and HLA-DRB3/4/5 alleles (as described in Paul, S., et al. Development and validation of a broad scheme for prediction of HLA class II restricted T cell epitopes. J Immunol Methods 422, 28-34 (2015), which is incorporated herein by reference) in order to identify the presence of T cell epitopes in NZM. The reference set of major histocompatibility complex (MHC) class II molecules included DRB1^{∗}13/14 that were found to be associated to NZM-related allergic reactions (de la Hera, B., et al. (2014) Natalizumab-related anaphylactoid reactions in MS patients are associated with HLA class II alleles. Neurol Neuroimmunol Neuroinflamm 1, e47). In particular, this study (de la Hera et al., 2014) reported a positive association of NZM-related allergic reactions with DRB1^{∗}13 and 14 alleles, suggesting a critical role for presentation of NZM peptides on MHC class II molecules and T-cell dependent B cell activation for the production of ADAs to NZM.

Results are shown in Figure 1. The data show that NetMHCIIpan 3.2 algorithm identified the LCDR2 epitope as the main peptide binding to MHC molecules with high-affinity, suggesting its potential immunodominance also in individuals with a diverse MHC background. Collectively, these findings demonstrate that only one of the potentially immunogenic peptides encoded by the six CDRs of NZM is a naturally presented T cell epitope with the potential to generate a T-cell response that helps B cells to produce anti-drug antibodies (ADAs) to NZM. In summary, a single immunodominant T cell epitope binding with high-affinity to several MHC-II molecules was identified, which is located in the CDR2 of NZM light chain (LCDR2).

### Example 2: Interaction of natalizumab (NZM) with α4 integrins

Next, the interaction of NZM with α4 integrins was studied to identify the role of the T cell epitope of NZM, which binds with high affinity to MHC molecules in α4 integrin binding.

To identify the residues that are important for the binding of NZM to α4-integrin, the structure of the NZM-α4β7 integrin complex (4IRZ) available in the Protein Data Bank (PDB) database (H.M. Berman, J. Westbrook, Z. Feng, G. Gilliland, T.N. Bhat, H. Weissig, I.N. Shindyalov, P.E. Bourne (2000) The Protein Data Bank Nucleic Acids Research, 28: 235-242; URL: https://www.rcsb.org/) was analyzed.

Several residues of the six CDRs of NZM were identified that were positioned at less than 4.0 Å distance from α4-integrin (Figure 2; Yu, Y., Schurpf, T. & Springer, T.A. How natalizumab binds and antagonizes alpha4 integrins. J Biol Chem 288, 32314-32325 (2013)). Interestingly, the LCDR2 contained only two residues interacting with α4-integrin. Those two interacting residues of NZM LCDR2 are positioned at the far ends of the loop, while the internal residues of the NZM LCDR2 are not required for α4-integrin binding. This analysis shows that the LCDR2 of NZM can be mutagenized without losing binding to α4-integrin. Accordingly, the LCDR2 of NZM contains residues that are not strictly required for the interaction with α4 integrins. In summary, structural analysis of NZM in complex to α4 integrin revealed the presence of few contact residues of NZM LCDR2 at the interface between the two molecules that are not crucial for binding to α4 integrin.

### Example 3: Design and construction of NZM LCDR2 variants that retain binding to α4 integrin

In view of the identification of the immunodominant LCDR2 epitope of NZM, which binds to MHC molecules, as described in Example 1, "deimmunized" versions of NZM were developed by structure-guided design.

First, residues of NZM light chain CDR2 were identified that were not engaging α4 integrin binding (positioned at more than 4.0 Å distance) and different mutants were modelled with the constraint to preserve the conformation of the CDR2 and the specificity of NZM. Four NZM variants (var1-4) were selected together with a fifth variant (var5) in which the CDR2 of the light chain was reverted to the germline sequence of the human antibody scaffold (Figure 3):
The heavy chain amino acid sequence of all five variants NZM var1 - NZM var5 is identical with that of NZM (including its CDRs and VH; with HCDR1-3 having the amino acid sequences of SEQ ID NOs 1 - 3, respectively; VH having the amino acid sequence of SEQ ID NO: 15; and the complete heavy chain having the amino acid sequence of SEQ ID NO: 25). The light chain amino acid sequence of NZM variants var1-4 differ from that of NZM only in the sequence of the LCDR2. NZM var5 was designed such that CDR2 of the light chain was reverted to the germline sequence of the human antibody scaffold. This was achieved by aligning the sequences of the NZM light chain with that of the human antibodies (REI) used for humanization (GenBank accession number 751419A). Therefore, in the case of var5, also the framework regions adjacent to LCDR2 are distinct. The CDR1 and CDR3 of the light chain are identical to those of NZM for all five variants (with LCDR1 and 3 having the amino acid sequences of SEQ ID NOs 4 and 6, respectively).

The LCDR2 of NZM has an amino acid sequence as set forth in SEQ ID NO: 11; the VL of NZM has an amino acid sequence as set forth in SEQ ID NO: 21; and the light chain of NZM has an amino acid sequence as set forth in SEQ ID NO: 31. The LCDR2 of NZM var1 has an amino acid sequence as set forth in SEQ ID NO: 9; the VL of NZM var1 has an amino acid sequence as set forth in SEQ ID NO: 19; and the light chain of NZM var1 has an amino acid sequence as set forth in SEQ ID NO: 29. The LCDR2 of NZM var2 has an amino acid sequence as set forth in SEQ ID NO: 12; the VL of NZM var2 has an amino acid sequence as set forth in SEQ ID NO: 22; and the light chain of NZM var2 has an amino acid sequence as set forth in SEQ ID NO: 32. The LCDR2 of NZM var3 has an amino acid sequence as set forth in SEQ ID NO: 10; the VL of NZM var3 has an amino acid sequence as set forth in SEQ ID NO: 20; and the light chain of NZM var3 has an amino acid sequence as set forth in SEQ ID NO: 30. The LCDR2 of NZM var4 has an amino acid sequence as set forth in SEQ ID NO: 13; the VL of NZM var4 has an amino acid sequence as set forth in SEQ ID NO: 23; and the light chain of NZM var4 has an amino acid sequence as set forth in SEQ ID NO: 33. The LCDR2 of NZM var5 has an amino acid sequence as set forth in SEQ ID NO: 14; the VL of NZM var5 has an amino acid sequence as set forth in SEQ ID NO: 24; and the light chain of NZM var5 has an amino acid sequence as set forth in SEQ ID NO: 34. Accordingly, NZM variant antibodies NZM var1 and NZM var3 represent exemplary antibodies of the invention, while NZM and variant antibodies NZM var2, NZM var4 and NZM var5 represent comparative antibodies.

A comparison of the amino acid sequences of the LCDR2 and the adjacent FR sections of NZM and variant antibodies NZM variants 1, 2, 3, 4 and 5 (NZM var1-5) is shown in Figure 3a.

NZM variants 1, 2, 3, 4 and 5 were expressed as IgG4 molecules and tested together with NZM for binding to α4 integrins by FACS. Briefly, synthetic genes expressing the NZM heavy chain and light chain variable regions (KEGG DRUG Database entry: D06886) were produced by Genscript and sublconed into vectors for expression of human IgG4 and human Igκ, respectively. NZM variants 1, 2, 3, 4 and 5 were synthetized by Genscript and subcloned into vectors for expression of full human IgG4. The chains were expressed following transient transfection of these vectors into Expi293F cells (ThermoFisher Scientific) using polyethylenimine.

To test binding to α4 integrins, serial dilutions of NZM and NZM variants were prepared in MACS buffer (PBS 1% FBS, 2mM EDTA). T cells isolated from healthy donors were used as source of α4-integrin and added (50,000 cell/well) to the plates for 30 min, 4°C. T cells were washed and stained with 3.75 µg/ml Alexa Fluor 647-conjugated goat anti-human IgG (Jackson ImmunoResearch, cat. no. 109-606-170) for 30 min, 4°C. Cells were washed and analyzed by FACS (iQue, Intellicyt). NZM binding was calculated as percentage of IgG⁺ stained cells.

Results are shown in Figure 3b. The analysis revealed that the two antibodies of the invention, NZM var1 and NZM var3, retained binding to α4 integrins, while comparative antibodies NZM var2 and NZM var4 showed partial loss of binding that was considerably reduced in the case of comparative antibody NZM var5. In summary, these data show that the functionality of NZM is maintained in the antibodies of the present invention.

### Example 4: Antibodies of the invention show reduced binding to MHC molecules

Next, the immunogenicity of the NZM variant antibodies described in Example 3 was investigated. To this end, the five NZM variants were analyzed whether the mutated LCDR2 sequences contained a potential T cell epitope as observed for wild type NZM (as described in Example 1) by using the NetMHCIIpan 3.2 server as described in Example 1.

Results are shown in Figure 4. Prediction of peptide binding to a reference set of nine DRB1 and DRB3/4/5 alleles showed a drastic reduction of binding affinity for the peptides of the antibodies of the invention NZM var1 and NZM var3 compared to the peptides of the original NZM antibody, while the peptides of comparative antibodies NZM var2 and NZM var4 showed an increased binding (Figure 4). In particular, NZM var1 showed the lowest predicted binding to MHC-II molecules. Comparative antibody NZM var5 had a similar binding compared to wild type NZM. Collectively, these results show that the exemplary antibodies of the invention NZM var1 and NZM var3 contain a LCDR2 sequence that is not expected to activate a T cell response. Accordingly, the antibodies of the invention represent "deimmunzed" versions of NZM.

**TABLE OF SEQUENCES AND SEQ ID NUMBERS (SEQUENCE LISTING)*:**

| SEQ ID NO | Sequence | Remarks |
|---|---|---|
| SEQ ID NO: 1 | GFNIKDTYIH | HCDR1 |
| SEQ ID NO: 2 | RIDPANGYTKYDPK | HCDR2 |
| SEQ ID NO: 3 | EGYYGNYGVYAMDY | HCDR3 |
| SEQ ID NO: 4 | KTSQDINKYMA | LCDR1 |
| SEQ ID NO: 5 | YX₁X₂X₃X₄X₅P | LCDR2 |
| | wherein | |
| | X₁ may be any amino acid, however, if X₂ is S, X₃ is A, X₄ is L and X₅ is Q, then X₁ is not T; | |
| | X₂ may be any amino acid, however, if X₁ is T, X₃ is A, X₄ is L and X₅ is Q, then X₂ is not S; | |
| | X₃ may be any amino acid, however, if X₁ is T, X₂ is S, X₄ is L and X₅ is Q, then X₃ is not A; | |
| | X₄ may be any amino acid, however, if X₁ is T, X₂ is S, X₃ is A and X₅ is Q, then X₄ is not L; and | |
| | X₅ may be any amino acid, however, if X₁ is T, X₂ is S, X₃ is A and X₄ is L, then X₅ is not Q | |
| SEQ ID NO: 6 | LQYDNLWT | LCDR3 |
| SEQ ID NO: 7 | YTX₂X₃X₄QP | LCDR2 |
| | wherein | |
| | X₂ may be any amino acid, however, if X₃ is A and X₄ is L, then X₂ is not S; | |
| | X₃ may be any amino acid, however, if X₂ is S and X₄ is L, then X₃ is not A; and | |
| | X₄ may be any amino acid, however, if X₂ is S and X₃ is A, then X₄ is not L | |
| SEQ ID NO: 8 | YTKX₃NQP | LCDR2 |
| | wherein | |
| | X₃ may be any amino acid | |
| SEQ ID NO: 9 | YTKGNQP | LCDR2 NZM var1 |
| SEQ ID NO: 10 | YTKSNQP | LCDR2 NZM var3 |
| SEQ ID NO: 11 | YTSALQP | LCDR2 NZM |
| SEQ ID NO: 12 | YTKGNQS | LCDR2 NZM var2 |
| SEQ ID NO: 13 | YTKSNQS | LCDR2 NZM var4 |
| SEQ ID NO: 14 | EASNLQA | LCDR2 NZM var5 |
| SEQ ID NO: 15 | | VH |
| SEQ ID NO: 16 | DIQMTQSPSSLSASVGDRVTITCKTSQDINKYMA WYQQTPGKAPRLLIHYX1X2X3X4X5PGIPSRFSGS GSGRDYTFTISSLQPEDIATYYCLQYDNLWTFG QGTKVEIK | VL |
| | wherein | |
| | X₁ may be any amino acid, however, if X₂ is S, X₃ is A, X₄ is L and X₅ is Q, then X₁ is not T; | |
| | X₂ may be any amino acid, however, if X₁ is T, X₃ is A, X₄ is L and X₅ is Q, then X₂ is not S; | |
| | X₃ may be any amino acid, however, if X₁ is T, X₂ is S, X₄ is L and X₅ is Q, then X₃ is not A; | |
| | X₄ may be any amino acid, however, if X₁ is T, X₂ is S, X₃ is A and X₅ is Q, then X₄ is not L; and | |
| | X₅ may be any amino acid, however, if X₁ is T, X₂ is S, X₃ is A and X₄ is L, then X₅ is not Q | |
| SEQ ID NO: 17 | DIQMTQSPSSLSASVGDRVTITCKTSQDINKYMA WYQQTPGKAPRLLIHYTX₂X₃X₄QPGIPSRFSGSG SGRDYTFTISSLQPEDIATYYCLQYDNLWTFGQ GTKVEIK | VL |
| | wherein | |
| | X₂ may be any amino acid, however, if X₃ is A and X₄ is L, then X₂ is not S; | |
| | X₃ may be any amino acid, however, if X₂ is S and X₄ is L, then X₃ is not A; and | |
| | X₄ may be any amino acid, however, if X₂ is S and X₃ is A, then X₄ is not L | |
| SEQ ID NO: 18 | DIQMTQSPSSLSASVGDRVTITCKTSQDINKYMA WYQQTPGKAPRLLIHYTKX3NQPGIPSRFSGSGS GRDYTFTISSLQPEDIATYYCLQYDNLWTFGQG TKVEIK | VL |
| | wherein | |
| | X₃ may be any amino acid | |
| SEQ ID NO: 19 | DIQMTQSPSSLSASVGDRVTITCKTSQDINKYMA WYQQTPGKAPRLLIHYTKGNQPGIPSRFSGSGS GRDYTFTISSLQPEDIATYYCLQYDNLWTFGQG TKVEIK | VL NZM var1 |
| SEQ ID NO: 20 | DIQMTQSPSSLSASVGDRVTITCKTSQDINKYMA WYQQTPGKAPRLLIHYTKSNQPGIPSRFSGSGS GRDYTFTISSLQPEDIATYYCLQYDNLWTFGQG TKVEIK | VL NZM var3 |
| SEQ ID NO: 21 | | VL NZM |
| SEQ ID NO: 22 | DIQMTQSPSSLSASVGDRVTITCKTSQDINKYMA WYQQTPGKAPRLLIHYTKGNQSGIPSRFSGSGS GRDYTFTISSLQPEDIATYYCLQYDNLWTFGQG TKVEIK | VL NZM var2 |
| SEQ ID NO: 23 | DIQMTQSPSSLSASVGDRVTITCKTSQDINKYMA WYQQTPGKAPRLLIHYTKSNQSGIPSRFSGSGSG RDYTFTISSLQPEDIATYYCLQYDNLWTFGQGTK VEIK | VL NZM var4 |
| SEQ ID NO: 24 | DIQMTQSPSSLSASVGDRVTITCKTSQDINKYMA WYQQTPGKAPRLLIHYTKSNQSGIPSRFSGSGSG RDYTFTISSLQPEDIATYYCLQYDNLWTFGQGTK VEIK | VL NZM var5 |
| SEQ ID NO: 25 | QVQLVQSGAEVKKPGASVKVSCKASGFNIKDTY IHWVRQAPGQRLEWMGRIDPANGYTKYDPKF QGRVTITADTSASTAYMELSSLRSEDTAVYYCARE GYYGNYGVYAMDYWGQGTLVTVSSASTKGPS VFPLAPCSRSTSESTAALGCLVKDYFPEPVTVSWN SGALTSGVHTFPAVLQSSGLYSLSSVVTVPSSSLG TKTYTCNVDHKPSNTKVDKRVESKYGPPCPSCP APEFLGGPSVFLFPPKPKDTLMISRTPEVTCVVVD VSQEDPEVQFNWYVDGVEVHNAKTKPREEQFN STYRVVSVLTVLHQDWLNGKEYKCKVSNKGLPS SIEKTISKAKGQPREPQVYTLPPSQEEMTKNQVSL TCLVKGFYPSDIAVEWESNGQPENNYKTTPPVL DSDGSFFLYSRLTVDKSRWQEGNVFSCSVMHEA LHNHYTQKSLSLSLGK | Heavy chain |
| SEQ ID NO: 26 | DIQMTQSPSSLSASVGDRVTITCKTSQDINKYMA WYQQTPGKAPRLLIHYX1X2X3X4X5PGIPSRFSGS GSGRDYTFTISSLQPEDIATYYCLQYDNLWTFG QGTKVEIKRTVAAPSVFIFPPSDEQLKSGTASVVC LLNNFYPREAKVQWKVDNALQSGNSQESVTEQ DSKDSTYSLSSTLTLSKADYEKHKVYACEVTHQG LSSPVTKSFNRGEC | Light chain |
| | wherein | |
| | X₁ may be any amino acid, however, if X₂ is S, X₃ is A, X₄ is L and X₅ is Q, then X₁ is not T; | |
| | X₂ may be any amino acid, however, if X₁ is T, X₃ is A, X₄ is L and X₅ is Q, then X₂ is not S; | |
| | X₃ may be any amino acid, however, if X₁ is T, X₂ is S, X₄ is L and X₅ is Q, then X₃ is not A; | |
| | X₄ may be any amino acid, however, if X₁ is T, X₂ is S, X₃ is A and X₅ is Q, then X₄ is not L; and | |
| | X₅ may be any amino acid, however, if X₁ is T, X₂ is S, X₃ is A and X₄ is L, then X₅ is not Q | |
| SEQ ID NO: 27 | DIQMTQSPSSLSASVGDRVTITCKTSQDINKYMA WYQQTPGKAPRLLIHYTX2X3X4QPGIPSRFSGSG SGRDYTFTISSLQPEDIATYYCLQYDNLWTFGQ GTKVEIKRTVAAPSVFIFPPSDEQLKSGTASVVCLL NNFYPREAKVQWKVDNALQSGNSQESVTEQD SKDSTYSLSSTLTLSKADYEKHKVYACEVTHQGLS SPVTKSFNRGEC | Light chain |
| | wherein | |
| | X₂ may be any amino acid, however, if X₃ is A and X₄ is L, then X₂ is not S; | |
| | X₃ may be any amino acid, however, if X₂ is S and X₄ is L, then X₃ is not A; and | |
| | X₄ may be any amino acid, however, if X₂ is S and X₃ is A, then X₄ is not L | |
| SEQ ID NO: 28 | DIQMTQSPSSLSASVGDRVTITCKTSQDINKYMA WYQQTPGKAPRLLIHYTKX3NQPGIPSRFSGSG SGRDYTFTISSLQPEDIATYYCLQYDNLWTFGQ GTKVEIKRTVAAPSVFIFPPSDEQLKSGTASVVCLL NNFYPREAKVQWKVDNALQSGNSQESVTEQD SKDSTYSLSSTLTLSKADYEKHKVYACEVTHQGLS SPVTKSFNRGEC | Light chain |
| | wherein | |
| | X₃ may be any amino acid | |
| SEQ ID NO: 29 | DIQMTQSPSSLSASVGDRVTITCKTSQDINKYMA WYQQTPGKAPRLLIHYTKGNQPGIPSRFSGSGS GRDYTFTISSLQPEDIATYYCLQYDNLWTFGQG TKVEIKRTVAAPSVFIFPPSDEQLKSGTASVVCLLN NFYPREAKVQWKVDNALQSGNSQESVTEQDSK DSTYSLSSTLTLSKADYEKHKVYACEVTHQGLSSP VTKSFNRGEC | Light chain NZM var1 |
| SEQ ID NO: 30 | DIQMTQSPSSLSASVGDRVTITCKTSQDINKYMA WYQQTPGKAPRLLIHYTKSNQPGIPSRFSGSGS GRDYTFTISSLQPEDIATYYCLQYDNLWTFGQG TKVEIKRTVAAPSVFIFPPSDEQLKSGTASVVCLLN NFYPREAKVQWKVDNALQSGNSQESVTEQDSK DSTYSLSSTLTLSKADYEKHKVYACEVTHQGLSSP VTKSFNRGEC | Light chain NZM var3 |
| SEQ ID NO: 31 | DIQMTQSPSSLSASVGDRVTITCKTSQDINKYMA WYQQTPGKAPRLLIHYTSALQPGIPSRFSGSGSG RDYTFTISSLQPEDIATYYCLQYDNLWTFGQGTK VEIKRTVAAPSVFIFPPSDEQLKSGTASVVCLLNNF YPREAKVQWKVDNALQSGNSQESVTEQDSKDS TYSLSSTLTLSKADYEKHKVYACEVTHQGLSSPVT KSFNRGEC | Light chain NZM |
| SEQ ID NO: 32 | DIQMTQSPSSLSASVGDRVTITCKTSQDINKYMA WYQQTPGKAPRLLIHYTKGNQSGIPSRFSGSGS GRDYTFTISSLQPEDIATYYCLQYDNLWTFGQG TKVEIKRTVAAPSVFIFPPSDEQLKSGTASVVCLLN NFYPREAKVQWKVDNALQSGNSQESVTEQDSK DSTYSLSSTLTLSKADYEKHKVYACEVTHQGLSSP VTKSFNRGEC | Light chain NZM var2 |
| SEQ ID NO: 33 | DIQMTQSPSSLSASVGDRVTITCKTSQDINKYMA WYQQTPGKAPRLLIHYTKSNQSGIPSRFSGSGSG RDYTFTISSLQPEDIATYYCLQYDNLWTFGQGTK VEIKRTVAAPSVFIFPPSDEQLKSGTASVVCLLNNF YPREAKVQWKVDNALQSGNSQESVTEQDSKDS TYSLSSTLTLSKADYEKHKVYACEVTHQGLSSPVT KSFNRGEC | Light chain NZM var4 |
| SEQ ID NO: 34 | DIQMTQSPSSLSASVGDRVTITCKTSQDINKYMA WYQQTPGKAPKLLIYEASNLQAGVPSRFSGSGS GRDYTFTISSLQPEDIATYYCLQYDNLWTFGQG TKVEIKRTVAAPSVFIFPPSDEQLKSGTASVVCLLN NFYPREAKVQWKVDNALQSGNSQESVTEQDSK DSTYSLSSTLTLSKADYEKHKVYACEVTHQGLSSP VTKSFNRGEC | Light chain NZM var5 |

| Constant regions | | |
|---|---|---|
| SEQ ID NO: 35 | ASTKGPSVFPLAPCSRSTSESTAALGCLVKDYFPE PVTVSWNSGALTSGVHTFPAVLQSSGLYSLSSVV TVPSSSLGTKTYTCNVDHKPSNTKVDKRVESKY GPPCPSCPAPEFLGGPSVFLFPPKPKDTLMISRTPE VTCVVVDVSQEDPEVQFNWYVDGVEVHNAKT KPREEQFNSTYRVVSVLTVLHQDWLNGKEYKCK VSNKGLPSSIEKTISKAKGQPREPQVYTLPPSQEE MTKNQVSLTCLVKGFYPSDIAVEWESNGQPEN NYKTTPPVLDSDGSFFLYSRLTVDKSRWQEGNV FSCSVMHEALHNHYTQKSLSLSLGK | heavy chain |
| SEQ ID NO: 36 | | light chain |

| | | |
|---|---|---|
| *CDRa are shown in bold; constant regions are shown in italics. | | |

## Claims

1. An antibody, or an antigen-binding fragment thereof, comprising (i) the heavy chain CDR1, CDR2, and CDR3 amino acid sequences as set forth in SEQ ID NO: 1, SEQ ID NO: 2, and SEQ ID NO: 3, respectively, and the light chain CDR1, CDR2, and CDR3 amino acid sequences as set forth in SEQ ID NO: 4, SEQ ID NO: 5, and SEQ ID NO: 6, respectively.

2. The antibody, or an antigen-binding fragment thereof, according to claim 1, wherein the antibody, or the antigen-binding fragment thereof, comprises the light chain CDR2 (LCDR2) amino acid sequence as set forth in SEQ ID NO: 7.

3. The antibody, or an antigen-binding fragment thereof, according to claim 1 or 2, wherein (i) X₂ in SEQ ID NO: 5 or 7 is selected from G, T, V, K, D and N; and/or (ii) X₄ in SEQ ID NO: 5 or 7 is selected from N, Q, V, I and S.

4. The antibody, or an antigen-binding fragment thereof, according to any one of the previous claims, wherein the antibody, or the antigen-binding fragment thereof, comprises the light chain CDR2 (LCDR2) amino acid sequence as set forth in SEQ ID NO: 8.

5. The antibody, or an antigen-binding fragment thereof, according to any one of the previous claims, wherein the antibody, or the antigen-binding fragment thereof, comprises the light chain CDR2 (LCDR2) amino acid sequence as set forth in SEQ ID NO: 9 or 10.

6. The antibody, or an antigen-binding fragment thereof, according to any one of the previous claims, wherein the antibody, or the antigen-binding fragment thereof, comprises a heavy chain variable region (VH) comprising an amino acid sequence having at least 70% identity to SEQ ID NO: 15 and a light chain variable region (VL) comprising an amino acid sequence having at least 70% identity to SEQ ID NO: 16.

7. The antibody, or an antigen-binding fragment thereof, according to any one of the previous claims, wherein the antibody, or the antigen-binding fragment thereof, comprises a heavy chain variable region (VH) comprising the amino acid sequence as set forth in SEQ ID NO: 15 and a light chain variable region (VL) comprising the amino acid sequence as set forth in SEQ ID NO: 19 or 20.

8. The antibody, or an antigen-binding fragment thereof, according to any one of the previous claims, wherein the antibody, or the antigen-binding fragment thereof, binds to α4 integrin.

9. The antibody, or an antigen-binding fragment thereof, according to any one of the previous claims, wherein the binding affinity of the antibody, or the antigen-binding fragment thereof, to an MHC molecule is decreased in comparison to the binding affinity of natalizumab to the same MHC molecule.

10. The antibody, or an antigen-binding fragment thereof, according to any one of the previous claims, wherein the antibody, or the antigen-binding fragment thereof, is a monoclonal antibody, a purified antibody, a single-chain antibody, Fab, Fab', F(ab')2, Fv or scFv.

11. The antibody, or an antigen-binding fragment thereof, according to any one of the previous claims for use as a medicament.

12. A nucleic acid molecule or a combination of nucleic acid molecules comprising a polynucleotide encoding the antibody, or an antigen-binding fragment thereof, of any one of claims 1 - 10.

13. A vector comprising the nucleic acid molecule or the combination of nucleic acid molecules according to claim 12; or a combination of vectors comprising the combination of nucleic acid molecules according to claim 12.

14. A cell expressing the antibody, or an antigen-binding fragment thereof, according to any one of claims 1 - 10, or comprising the nucleic acid molecule or the combination of nucleic acid molecules according to claim 12 or the vector or the combination of vectors according to claim 13.

15. A pharmaceutical composition comprising the antibody, or an antigen-binding fragment thereof, according to any one of claims 1 - 10, the nucleic acid molecule or the combination or the combination of nucleic acid molecules according to claim 12, the vector or the combination of vectors according to claim 13, or the cell according to claim 14, and, optionally, a pharmaceutically acceptable diluent or carrier.


SEQUENCE LISTING

<110>  Institute for Research in Biomedicine

<120>  DEIMMUNIZED ANTIBODIES BINDING TO ALPHA-4 INTEGRIN AND USES
       THEREOF

<130>  IR01P013EP

<160>  36

<170>  PatentIn version 3.5

<210>  1
<211>  10
<212>  PRT
<213>  Artificial Sequence

<220>
<223>  HCDR1

<400>  1

Gly Phe Asn Ile Lys Asp Thr Tyr Ile His
1               5                   10


<210>  2
<211>  14
<212>  PRT
<213>  Artificial Sequence

<220>
<223>  HCDR2

<400>  2

Arg Ile Asp Pro Ala Asn Gly Tyr Thr Lys Tyr Asp Pro Lys
1               5                   10


<210>  3
<211>  14
<212>  PRT
<213>  Artificial Sequence

<220>
<223>  HCDR3

<400>  3

Glu Gly Tyr Tyr Gly Asn Tyr Gly Val Tyr Ala Met Asp Tyr
1               5                   10


<210>  4
<211>  11
<212>  PRT
<213>  Artificial Sequence

<220>
<223>  LCDR1

<400>  4

Lys Thr Ser Gln Asp Ile Asn Lys Tyr Met Ala
1               5                   10



<210>  5
<211>  7
<212>  PRT
<213>  Artificial Sequence

<220>
<223>  LCDR2



<220>
<221>  MISC_FEATURE
<222>  (2)..(2)
<223>  X1 may be any amino acid, however, if X2 is S, X3 is A, X4 is L
       and X5 is Q, then X1 is not T

<220>
<221>  MISC_FEATURE
<222>  (3)..(3)
<223>  X2 may be any amino acid, however, if X1 is T, X3 is A, X4 is L
       and X5 is Q, then X2 is not S

<220>
<221>  MISC_FEATURE
<222>  (4)..(4)
<223>  X3 may be any amino acid, however, if X1 is T, X2 is S, X4 is L
       and X5 is Q, then X3 is not A

<220>
<221>  MISC_FEATURE
<222>  (5)..(5)
<223>  X4 may be any amino acid, however, if X1 is T, X2 is S, X3 is A
       and X5 is Q, then X4 is not L

<220>
<221>  MISC_FEATURE
<222>  (6)..(6)
<223>  X5 may be any amino acid, however, if X1 is T, X2 is S, X3 is A
       and X4 is L, then X5 is not Q

<400>  5

Tyr Xaa Xaa Xaa Xaa Xaa Pro
1               5



<210>  6
<211>  8
<212>  PRT
<213>  Artificial Sequence

<220>
<223>  LCDR3

<400>  6

Leu Gln Tyr Asp Asn Leu Trp Thr
1               5

<210>  7
<211>  7
<212>  PRT
<213>  Artificial Sequence

<220>
<223>  LCDR2


<220>
<221>  MISC_FEATURE
<222>  (3)..(3)
<223>  X2 may be any amino acid, however, if X3 is A and X4 is L, then
       X2 is not S

<220>
<221>  MISC_FEATURE
<222>  (4)..(4)
<223>  X3 may be any amino acid, however, if X2 is S and X4 is L, then
       X3 is not A

<220>
<221>  MISC_FEATURE
<222>  (5)..(5)
<223>  X4 may be any amino acid, however, if X2 is S and X3 is A, then
       X4 is not L

<400>  7

Tyr Thr Xaa Xaa Xaa Gln Pro
1               5


<210>  8
<211>  7
<212>  PRT
<213>  Artificial Sequence

<220>
<223>  LCDR2


<220>
<221>  MISC_FEATURE
<222>  (4)..(4)
<223>  Xaa may be any amino acid

<400>  8

Tyr Thr Lys Xaa Asn Gln Pro
1               5


<210>  9
<211>  7
<212>  PRT
<213>  Artificial Sequence

<220>
<223>  LCDR2 NZM var1

<400>  9

Tyr Thr Lys Gly Asn Gln Pro
1               5


<210>  10
<211>  7
<212>  PRT
<213>  Artificial Sequence

<220>
<223>  LCDR2 NZM var3

<400>  10

Tyr Thr Lys Ser Asn Gln Pro
1               5


<210>  11
<211>  7
<212>  PRT
<213>  Artificial Sequence

<220>
<223>  LCDR2 NZM

<400>  11

Tyr Thr Ser Ala Leu Gln Pro
1               5


<210>  12
<211>  7
<212>  PRT
<213>  Artificial Sequence

<220>
<223>  LCDR2 NZM var2

<400>  12

Tyr Thr Lys Gly Asn Gln Ser
1               5


<210>  13
<211>  7
<212>  PRT
<213>  Artificial Sequence

<220>
<223>  LCDR2 NZM var4

<400>  13

Tyr Thr Lys Ser Asn Gln Ser
1               5


<210>  14

<211>  7
<212>  PRT
<213>  Artificial Sequence

<220>
<223>  LCDR2 NZM var5

<400>  14

Glu Ala Ser Asn Leu Gln Ala
1               5


<210>  15
<211>  123
<212>  PRT
<213>  Artificial Sequence

<220>
<223>  VH

<400>  15


<210>  16
<211>  106
<212>  PRT
<213>  Artificial Sequence

<220>
<223>  VL

<220>
<221>  MISC_FEATURE
<222>  (51)..(51)
<223>  X1 may be any amino acid, however, if X2 is S, X3 is A, X4 is L
       and X5 is Q, then X1 is not T

<220>
<221>  MISC_FEATURE
<222>  (52)..(52)
<223>  X2 may be any amino acid, however, if X1 is T, X3 is A, X4 is L
       and X5 is Q, then X2 is not S

<220>
<221>  MISC_FEATURE
<222>  (53)..(53)
<223>  X3 may be any amino acid, however, if X1 is T, X2 is S, X4 is L
       and X5 is Q, then X3 is not A

<220>
<221>  MISC_FEATURE
<222>  (54)..(54)
<223>  X4 may be any amino acid, however, if X1 is T, X2 is S, X3 is A
       and X5 is Q, then X4 is not L

<220>
<221>  MISC_FEATURE
<222>  (55)..(55)
<223>  X5 may be any amino acid, however, if X1 is T, X2 is S, X3 is A
       and X4 is L, then X5 is not Q

<400>  16

Asp Ile Gln Met Thr Gln Ser Pro Ser Ser Leu Ser Ala Ser Val Gly
1               5                   10                  15

Asp Arg Val Thr Ile Thr Cys Lys Thr Ser Gln Asp Ile Asn Lys Tyr
            20                  25                  30

Met Ala Trp Tyr Gln Gln Thr Pro Gly Lys Ala Pro Arg Leu Leu Ile
        35                  40                  45

His Tyr Xaa Xaa Xaa Xaa Xaa Pro Gly Ile Pro Ser Arg Phe Ser Gly
    50                  55                  60

Ser Gly Ser Gly Arg Asp Tyr Thr Phe Thr Ile Ser Ser Leu Gln Pro
65                  70                  75                  80

Glu Asp Ile Ala Thr Tyr Tyr Cys Leu Gln Tyr Asp Asn Leu Trp Thr
                85                  90                  95

Phe Gly Gln Gly Thr Lys Val Glu Ile Lys
            100                 105

<210>  17
<211>  106

<212>  PRT
<213>  Artificial Sequence

<220>
<223>  VL



<220>
<221>  MISC_FEATURE
<222>  (52)..(52)
<223>  X2 may be any amino acid, however, if X3 is A and X4 is L, then
       X2 is not S

<220>
<221>  MISC_FEATURE
<222>  (53)..(53)
<223>  X3 may be any amino acid, however, if X2 is S and X4 is L, then
       X3 is not A

<220>
<221>  MISC_FEATURE
<222>  (54)..(54)
<223>  X4 may be any amino acid, however, if X2 is S and X3 is A, then
       X4 is not L

<400>  17

Asp Ile Gln Met Thr Gln Ser Pro Ser Ser Leu Ser Ala Ser Val Gly
1               5                   10                  15


Asp Arg Val Thr Ile Thr Cys Lys Thr Ser Gln Asp Ile Asn Lys Tyr
            20                  25                  30


Met Ala Trp Tyr Gln Gln Thr Pro Gly Lys Ala Pro Arg Leu Leu Ile
        35                  40                  45


His Tyr Thr Xaa Xaa Xaa Gln Pro Gly Ile Pro Ser Arg Phe Ser Gly
    50                  55                  60


Ser Gly Ser Gly Arg Asp Tyr Thr Phe Thr Ile Ser Ser Leu Gln Pro
65                  70                  75                  80


Glu Asp Ile Ala Thr Tyr Tyr Cys Leu Gln Tyr Asp Asn Leu Trp Thr
                85                  90                  95


Phe Gly Gln Gly Thr Lys Val Glu Ile Lys
            100                 105


<210>  18
<211>  106
<212>  PRT
<213>  Artificial Sequence

<220>
<223>  VL

<220>
<221>  MISC_FEATURE
<222>  (53)..(53)
<223>  Xaa may be any amino acid

<400>  18

Asp Ile Gln Met Thr Gln Ser Pro Ser Ser Leu Ser Ala Ser Val Gly
1               5                   10                  15

Asp Arg Val Thr Ile Thr Cys Lys Thr Ser Gln Asp Ile Asn Lys Tyr
            20                  25                  30

Met Ala Trp Tyr Gln Gln Thr Pro Gly Lys Ala Pro Arg Leu Leu Ile
        35                  40                  45

His Tyr Thr Lys Xaa Asn Gln Pro Gly Ile Pro Ser Arg Phe Ser Gly
    50                  55                  60

Ser Gly Ser Gly Arg Asp Tyr Thr Phe Thr Ile Ser Ser Leu Gln Pro
65                  70                  75                  80

Glu Asp Ile Ala Thr Tyr Tyr Cys Leu Gln Tyr Asp Asn Leu Trp Thr
                85                  90                  95

Phe Gly Gln Gly Thr Lys Val Glu Ile Lys
            100                 105


<210>  19
<211>  106
<212>  PRT
<213>  Artificial Sequence

<220>
<223>  VL NZM var1

<400>  19

Asp Ile Gln Met Thr Gln Ser Pro Ser Ser Leu Ser Ala Ser Val Gly
1               5                   10                  15

Asp Arg Val Thr Ile Thr Cys Lys Thr Ser Gln Asp Ile Asn Lys Tyr
            20                  25                  30

Met Ala Trp Tyr Gln Gln Thr Pro Gly Lys Ala Pro Arg Leu Leu Ile
        35                  40                  45

His Tyr Thr Lys Gly Asn Gln Pro Gly Ile Pro Ser Arg Phe Ser Gly
    50                  55                  60

Ser Gly Ser Gly Arg Asp Tyr Thr Phe Thr Ile Ser Ser Leu Gln Pro

65                  70                  75                  80

Glu Asp Ile Ala Thr Tyr Tyr Cys Leu Gln Tyr Asp Asn Leu Trp Thr
                85                  90                  95

Phe Gly Gln Gly Thr Lys Val Glu Ile Lys
            100                 105

<210>  20
<211>  106
<212>  PRT
<213>  Artificial Sequence

<220>
<223>  VL NZM var3

<400>  20

Asp Ile Gln Met Thr Gln Ser Pro Ser Ser Leu Ser Ala Ser Val Gly
1               5                   10                  15

Asp Arg Val Thr Ile Thr Cys Lys Thr Ser Gln Asp Ile Asn Lys Tyr
            20                  25                  30

Met Ala Trp Tyr Gln Gln Thr Pro Gly Lys Ala Pro Arg Leu Leu Ile
        35                  40                  45

His Tyr Thr Lys Ser Asn Gln Pro Gly Ile Pro Ser Arg Phe Ser Gly
    50                  55                  60

Ser Gly Ser Gly Arg Asp Tyr Thr Phe Thr Ile Ser Ser Leu Gln Pro
65                  70                  75                  80

Glu Asp Ile Ala Thr Tyr Tyr Cys Leu Gln Tyr Asp Asn Leu Trp Thr
                85                  90                  95

Phe Gly Gln Gly Thr Lys Val Glu Ile Lys
            100                 105

<210>  21
<211>  106
<212>  PRT
<213>  Artificial Sequence

<220>
<223>  VL NZM

<400>  21

<210> 22
<211> 106
<212> PRT
<213> Artificial Sequence

<220>
<223> VL NZM var2

<400> 22

Asp Ile Gln Met Thr Gln Ser Pro Ser Ser Leu Ser Ala Ser Val Gly
1               5                   10                  15

Asp Arg Val Thr Ile Thr Cys Lys Thr Ser Gln Asp Ile Asn Lys Tyr
            20                  25                  30

Met Ala Trp Tyr Gln Gln Thr Pro Gly Lys Ala Pro Arg Leu Leu Ile
        35                  40                  45

His Tyr Thr Lys Gly Asn Gln Ser Gly Ile Pro Ser Arg Phe Ser Gly
    50                  55                  60

Ser Gly Ser Gly Arg Asp Tyr Thr Phe Thr Ile Ser Ser Leu Gln Pro
65                  70                  75                  80

Glu Asp Ile Ala Thr Tyr Tyr Cys Leu Gln Tyr Asp Asn Leu Trp Thr
                85                  90                  95

Phe Gly Gln Gly Thr Lys Val Glu Ile Lys
            100                 105

<210> 23

<211>  106
<212>  PRT
<213>  Artificial Sequence

<220>
<223>  VL NZM var4

<400>  23

Asp Ile Gln Met Thr Gln Ser Pro Ser Ser Leu Ser Ala Ser Val Gly
1               5                   10                  15

Asp Arg Val Thr Ile Thr Cys Lys Thr Ser Gln Asp Ile Asn Lys Tyr
            20                  25                  30

Met Ala Trp Tyr Gln Gln Thr Pro Gly Lys Ala Pro Arg Leu Leu Ile
        35                  40                  45

His Tyr Thr Lys Ser Asn Gln Ser Gly Ile Pro Ser Arg Phe Ser Gly
    50                  55                  60

Ser Gly Ser Gly Arg Asp Tyr Thr Phe Thr Ile Ser Ser Leu Gln Pro
65                  70                  75                  80

Glu Asp Ile Ala Thr Tyr Tyr Cys Leu Gln Tyr Asp Asn Leu Trp Thr
                85                  90                  95

Phe Gly Gln Gly Thr Lys Val Glu Ile Lys
            100                 105


<210>  24
<211>  106
<212>  PRT
<213>  Artificial Sequence

<220>
<223>  VL NZM var5

<400>  24

Asp Ile Gln Met Thr Gln Ser Pro Ser Ser Leu Ser Ala Ser Val Gly
1               5                   10                  15

Asp Arg Val Thr Ile Thr Cys Lys Thr Ser Gln Asp Ile Asn Lys Tyr
            20                  25                  30

Met Ala Trp Tyr Gln Gln Thr Pro Gly Lys Ala Pro Arg Leu Leu Ile
        35                  40                  45

His Tyr Thr Lys Ser Asn Gln Ser Gly Ile Pro Ser Arg Phe Ser Gly
    50                  55                  60

Ser Gly Ser Gly Arg Asp Tyr Thr Phe Thr Ile Ser Ser Leu Gln Pro
65                  70                  75                  80

Glu Asp Ile Ala Thr Tyr Tyr Cys Leu Gln Tyr Asp Asn Leu Trp Thr
                85                  90                  95

Phe Gly Gln Gly Thr Lys Val Glu Ile Lys
            100                 105

<210>  25
<211>  450
<212>  PRT
<213>  Artificial Sequence

<220>
<223>  Heavy chain

<400>  25

Gln Val Gln Leu Val Gln Ser Gly Ala Glu Val Lys Lys Pro Gly Ala
1               5                   10                  15

Ser Val Lys Val Ser Cys Lys Ala Ser Gly Phe Asn Ile Lys Asp Thr
            20                  25                  30

Tyr Ile His Trp Val Arg Gln Ala Pro Gly Gln Arg Leu Glu Trp Met
        35                  40                  45

Gly Arg Ile Asp Pro Ala Asn Gly Tyr Thr Lys Tyr Asp Pro Lys Phe
    50                  55                  60

Gln Gly Arg Val Thr Ile Thr Ala Asp Thr Ser Ala Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Glu Gly Tyr Tyr Gly Asn Tyr Gly Val Tyr Ala Met Asp Tyr
            100                 105                 110

Trp Gly Gln Gly Thr Leu Val Thr Val Ser Ser Ala Ser Thr Lys Gly
        115                 120                 125

Pro Ser Val Phe Pro Leu Ala Pro Cys Ser Arg Ser Thr Ser Glu Ser
    130                 135                 140

Thr Ala Ala Leu Gly Cys Leu Val Lys Asp Tyr Phe Pro Glu Pro Val
145                 150                 155                 160

Thr Val Ser Trp Asn Ser Gly Ala Leu Thr Ser Gly Val His Thr Phe

                165                 170                 175

Pro Ala Val Leu Gln Ser Ser Gly Leu Tyr Ser Leu Ser Ser Val Val
            180                 185                 190

Thr Val Pro Ser Ser Ser Leu Gly Thr Lys Thr Tyr Thr Cys Asn Val
        195                 200                 205

Asp His Lys Pro Ser Asn Thr Lys Val Asp Lys Arg Val Glu Ser Lys
    210                 215                 220

Tyr Gly Pro Pro Cys Pro Ser Cys Pro Ala Pro Glu Phe Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser Gln Glu
            260                 265                 270

Asp Pro Glu Val Gln Phe Asn Trp Tyr Val Asp Gly Val Glu Val His
        275                 280                 285

Asn Ala Lys Thr Lys Pro Arg Glu Glu Gln Phe Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Gly Leu Pro Ser Ser Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Gln Glu Glu Met Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Arg Leu Thr Val Asp
                405                 410                 415

Lys Ser Arg Trp Gln Glu Gly Asn Val Phe Ser Cys Ser Val Met His
            420                 425                 430

Glu Ala Leu His Asn His Tyr Thr Gln Lys Ser Leu Ser Leu Ser Leu
        435                 440                 445

Gly Lys
    450

<210>  26
<211>  213
<212>  PRT
<213>  Artificial Sequence

<220>
<223>  Light chain

<220>
<221>  MISC_FEATURE
<222>  (51)..(51)
<223>  X1 may be any amino acid, however, if X2 is S, X3 is A, X4 is L
       and X5 is Q, then X1 is not T

<220>
<221>  MISC_FEATURE
<222>  (52)..(52)
<223>  X2 may be any amino acid, however, if X1 is T, X3 is A, X4 is L
       and X5 is Q, then X2 is not S

<220>
<221>  MISC_FEATURE
<222>  (53)..(53)
<223>  X3 may be any amino acid, however, if X1 is T, X2 is S, X4 is L
       and X5 is Q, then X3 is not A

<220>
<221>  MISC_FEATURE
<222>  (54)..(54)
<223>  X4 may be any amino acid, however, if X1 is T, X2 is S, X3 is A
       and X5 is Q, then X4 is not L

<220>
<221>  MISC_FEATURE
<222>  (55)..(55)
<223>  X5 may be any amino acid, however, if X1 is T, X2 is S, X3 is A
       and X4 is L, then X5 is not Q

<400>  26

Asp Ile Gln Met Thr Gln Ser Pro Ser Ser Leu Ser Ala Ser Val Gly
1               5                   10                  15

Asp Arg Val Thr Ile Thr Cys Lys Thr Ser Gln Asp Ile Asn Lys Tyr
            20                  25                  30

Met Ala Trp Tyr Gln Gln Thr Pro Gly Lys Ala Pro Arg Leu Leu Ile
        35                  40                  45

His Tyr Xaa Xaa Xaa Xaa Xaa Pro Gly Ile Pro Ser Arg Phe Ser Gly
    50                  55                  60

Ser Gly Ser Gly Arg Asp Tyr Thr Phe Thr Ile Ser Ser Leu Gln Pro
65                  70                  75                  80

Glu Asp Ile Ala Thr Tyr Tyr Cys Leu Gln Tyr Asp Asn Leu Trp Thr
                85                  90                  95

Phe Gly Gln Gly Thr Lys Val Glu Ile Lys Arg Thr Val Ala Ala Pro
            100                 105                 110

Ser Val Phe Ile Phe Pro Pro Ser Asp Glu Gln Leu Lys Ser Gly Thr
        115                 120                 125

Ala Ser Val Val Cys Leu Leu Asn Asn Phe Tyr Pro Arg Glu Ala Lys
    130                 135                 140

Val Gln Trp Lys Val Asp Asn Ala Leu Gln Ser Gly Asn Ser Gln Glu
145                 150                 155                 160

Ser Val Thr Glu Gln Asp Ser Lys Asp Ser Thr Tyr Ser Leu Ser Ser
                165                 170                 175

Thr Leu Thr Leu Ser Lys Ala Asp Tyr Glu Lys His Lys Val Tyr Ala
            180                 185                 190

Cys Glu Val Thr His Gln Gly Leu Ser Ser Pro Val Thr Lys Ser Phe
        195                 200                 205

Asn Arg Gly Glu Cys
    210

<210>  27
<211>  213
<212>  PRT
<213>  Artificial Sequence

<220>
<223>  Light chain

<220>
<221>  MISC_FEATURE
<222>  (52)..(52)
<223>  X2 may be any amino acid, however, if X3 is A and X4 is L, then
       X2 is not S

<220>
<221>  MISC_FEATURE

<222>  (53)..(53)
<223>  X3 may be any amino acid, however, if X2 is S and X4 is L, then
       X3 is not A

<220>
<221>  MISC_FEATURE
<222>  (54)..(54)
<223>  X4 may be any amino acid, however, if X2 is S and X3 is A, then
       X4 is not L

<400>  27

Asp Ile Gln Met Thr Gln Ser Pro Ser Ser Leu Ser Ala Ser Val Gly
1               5                   10                  15

Asp Arg Val Thr Ile Thr Cys Lys Thr Ser Gln Asp Ile Asn Lys Tyr
            20                  25                  30

Met Ala Trp Tyr Gln Gln Thr Pro Gly Lys Ala Pro Arg Leu Leu Ile
        35                  40                  45

His Tyr Thr Xaa Xaa Xaa Gln Pro Gly Ile Pro Ser Arg Phe Ser Gly
    50                  55                  60

Ser Gly Ser Gly Arg Asp Tyr Thr Phe Thr Ile Ser Ser Leu Gln Pro
65                  70                  75                  80

Glu Asp Ile Ala Thr Tyr Tyr Cys Leu Gln Tyr Asp Asn Leu Trp Thr
                85                  90                  95

Phe Gly Gln Gly Thr Lys Val Glu Ile Lys Arg Thr Val Ala Ala Pro
            100                 105                 110

Ser Val Phe Ile Phe Pro Pro Ser Asp Glu Gln Leu Lys Ser Gly Thr
        115                 120                 125

Ala Ser Val Val Cys Leu Leu Asn Asn Phe Tyr Pro Arg Glu Ala Lys
    130                 135                 140

Val Gln Trp Lys Val Asp Asn Ala Leu Gln Ser Gly Asn Ser Gln Glu
145                 150                 155                 160

Ser Val Thr Glu Gln Asp Ser Lys Asp Ser Thr Tyr Ser Leu Ser Ser
                165                 170                 175

Thr Leu Thr Leu Ser Lys Ala Asp Tyr Glu Lys His Lys Val Tyr Ala
            180                 185                 190

Cys Glu Val Thr His Gln Gly Leu Ser Ser Pro Val Thr Lys Ser Phe
        195                 200                 205

Asn Arg Gly Glu Cys
    210

<210>  28
<211>  213
<212>  PRT
<213>  Artificial Sequence

<220>
<223>  Light chain

<220>
<221>  MISC_FEATURE
<222>  (53)..(53)
<223>  Xaa may be any amino acid

<400>  28

Asp Ile Gln Met Thr Gln Ser Pro Ser Ser Leu Ser Ala Ser Val Gly
1               5                   10                  15

Asp Arg Val Thr Ile Thr Cys Lys Thr Ser Gln Asp Ile Asn Lys Tyr
            20                  25                  30

Met Ala Trp Tyr Gln Gln Thr Pro Gly Lys Ala Pro Arg Leu Leu Ile
        35                  40                  45

His Tyr Thr Lys Xaa Asn Gln Pro Gly Ile Pro Ser Arg Phe Ser Gly
    50                  55                  60

Ser Gly Ser Gly Arg Asp Tyr Thr Phe Thr Ile Ser Ser Leu Gln Pro
65                  70                  75                  80

Glu Asp Ile Ala Thr Tyr Tyr Cys Leu Gln Tyr Asp Asn Leu Trp Thr
                85                  90                  95

Phe Gly Gln Gly Thr Lys Val Glu Ile Lys Arg Thr Val Ala Ala Pro
            100                 105                 110

Ser Val Phe Ile Phe Pro Pro Ser Asp Glu Gln Leu Lys Ser Gly Thr
        115                 120                 125

Ala Ser Val Val Cys Leu Leu Asn Asn Phe Tyr Pro Arg Glu Ala Lys
    130                 135                 140

Val Gln Trp Lys Val Asp Asn Ala Leu Gln Ser Gly Asn Ser Gln Glu
145                 150                 155                 160

Ser Val Thr Glu Gln Asp Ser Lys Asp Ser Thr Tyr Ser Leu Ser Ser
                165                 170                 175

Thr Leu Thr Leu Ser Lys Ala Asp Tyr Glu Lys His Lys Val Tyr Ala
            180                 185                 190

Cys Glu Val Thr His Gln Gly Leu Ser Ser Pro Val Thr Lys Ser Phe
        195                 200                 205

Asn Arg Gly Glu Cys
    210

<210>  29
<211>  213
<212>  PRT
<213>  Artificial Sequence

<220>
<223>  Light chain NZM var1

<400>  29

Asp Ile Gln Met Thr Gln Ser Pro Ser Ser Leu Ser Ala Ser Val Gly
1               5                   10                  15

Asp Arg Val Thr Ile Thr Cys Lys Thr Ser Gln Asp Ile Asn Lys Tyr
            20                  25                  30

Met Ala Trp Tyr Gln Gln Thr Pro Gly Lys Ala Pro Arg Leu Leu Ile
        35                  40                  45

His Tyr Thr Lys Gly Asn Gln Pro Gly Ile Pro Ser Arg Phe Ser Gly
    50                  55                  60

Ser Gly Ser Gly Arg Asp Tyr Thr Phe Thr Ile Ser Ser Leu Gln Pro
65                  70                  75                  80

Glu Asp Ile Ala Thr Tyr Tyr Cys Leu Gln Tyr Asp Asn Leu Trp Thr
                85                  90                  95

Phe Gly Gln Gly Thr Lys Val Glu Ile Lys Arg Thr Val Ala Ala Pro
            100                 105                 110

Ser Val Phe Ile Phe Pro Pro Ser Asp Glu Gln Leu Lys Ser Gly Thr
        115                 120                 125

Ala Ser Val Val Cys Leu Leu Asn Asn Phe Tyr Pro Arg Glu Ala Lys
    130                 135                 140

Val Gln Trp Lys Val Asp Asn Ala Leu Gln Ser Gly Asn Ser Gln Glu
145                 150                 155                 160

Ser Val Thr Glu Gln Asp Ser Lys Asp Ser Thr Tyr Ser Leu Ser Ser
                165                 170                 175

Thr Leu Thr Leu Ser Lys Ala Asp Tyr Glu Lys His Lys Val Tyr Ala
            180                 185                 190

Cys Glu Val Thr His Gln Gly Leu Ser Ser Pro Val Thr Lys Ser Phe
        195                 200                 205

Asn Arg Gly Glu Cys
    210

<210>  30
<211>  213
<212>  PRT
<213>  Artificial Sequence

<220>
<223>  Light chain NZM var3

<400>  30

Asp Ile Gln Met Thr Gln Ser Pro Ser Ser Leu Ser Ala Ser Val Gly
1               5                   10                  15

Asp Arg Val Thr Ile Thr Cys Lys Thr Ser Gln Asp Ile Asn Lys Tyr
            20                  25                  30

Met Ala Trp Tyr Gln Gln Thr Pro Gly Lys Ala Pro Arg Leu Leu Ile
        35                  40                  45

His Tyr Thr Lys Ser Asn Gln Pro Gly Ile Pro Ser Arg Phe Ser Gly
    50                  55                  60

Ser Gly Ser Gly Arg Asp Tyr Thr Phe Thr Ile Ser Ser Leu Gln Pro
65                  70                  75                  80

Glu Asp Ile Ala Thr Tyr Tyr Cys Leu Gln Tyr Asp Asn Leu Trp Thr
                85                  90                  95

Phe Gly Gln Gly Thr Lys Val Glu Ile Lys Arg Thr Val Ala Ala Pro
            100                 105                 110

Ser Val Phe Ile Phe Pro Pro Ser Asp Glu Gln Leu Lys Ser Gly Thr
        115                 120                 125

Ala Ser Val Val Cys Leu Leu Asn Asn Phe Tyr Pro Arg Glu Ala Lys
    130                 135                 140

Val Gln Trp Lys Val Asp Asn Ala Leu Gln Ser Gly Asn Ser Gln Glu
145                 150                 155                 160

Ser Val Thr Glu Gln Asp Ser Lys Asp Ser Thr Tyr Ser Leu Ser Ser
                165                 170                 175

Thr Leu Thr Leu Ser Lys Ala Asp Tyr Glu Lys His Lys Val Tyr Ala
            180                 185                 190

Cys Glu Val Thr His Gln Gly Leu Ser Ser Pro Val Thr Lys Ser Phe
        195                 200                 205

Asn Arg Gly Glu Cys
    210

<210>  31
<211>  213
<212>  PRT
<213>  Artificial Sequence

<220>
<223>  Light chain NZM

<400>  31

Asp Ile Gln Met Thr Gln Ser Pro Ser Ser Leu Ser Ala Ser Val Gly
1               5                   10                  15

Asp Arg Val Thr Ile Thr Cys Lys Thr Ser Gln Asp Ile Asn Lys Tyr
            20                  25                  30

Met Ala Trp Tyr Gln Gln Thr Pro Gly Lys Ala Pro Arg Leu Leu Ile
        35                  40                  45

His Tyr Thr Ser Ala Leu Gln Pro Gly Ile Pro Ser Arg Phe Ser Gly
    50                  55                  60

Ser Gly Ser Gly Arg Asp Tyr Thr Phe Thr Ile Ser Ser Leu Gln Pro
65                  70                  75                  80

Glu Asp Ile Ala Thr Tyr Tyr Cys Leu Gln Tyr Asp Asn Leu Trp Thr
                85                  90                  95

Phe Gly Gln Gly Thr Lys Val Glu Ile Lys Arg Thr Val Ala Ala Pro
            100                 105                 110

Ser Val Phe Ile Phe Pro Pro Ser Asp Glu Gln Leu Lys Ser Gly Thr
        115                 120                 125

Ala Ser Val Val Cys Leu Leu Asn Asn Phe Tyr Pro Arg Glu Ala Lys

130                 135                 140

Val Gln Trp Lys Val Asp Asn Ala Leu Gln Ser Gly Asn Ser Gln Glu
145                 150                 155                 160

Ser Val Thr Glu Gln Asp Ser Lys Asp Ser Thr Tyr Ser Leu Ser Ser
                165                 170                 175

Thr Leu Thr Leu Ser Lys Ala Asp Tyr Glu Lys His Lys Val Tyr Ala
            180                 185                 190

Cys Glu Val Thr His Gln Gly Leu Ser Ser Pro Val Thr Lys Ser Phe
        195                 200                 205

Asn Arg Gly Glu Cys
    210

<210>  32
<211>  213
<212>  PRT
<213>  Artificial Sequence

<220>
<223>  Light chain NZM var2

<400>  32

Asp Ile Gln Met Thr Gln Ser Pro Ser Ser Leu Ser Ala Ser Val Gly
1               5                   10                  15

Asp Arg Val Thr Ile Thr Cys Lys Thr Ser Gln Asp Ile Asn Lys Tyr
            20                  25                  30

Met Ala Trp Tyr Gln Gln Thr Pro Gly Lys Ala Pro Arg Leu Leu Ile
        35                  40                  45

His Tyr Thr Lys Gly Asn Gln Ser Gly Ile Pro Ser Arg Phe Ser Gly
    50                  55                  60

Ser Gly Ser Gly Arg Asp Tyr Thr Phe Thr Ile Ser Ser Leu Gln Pro
65                  70                  75                  80

Glu Asp Ile Ala Thr Tyr Tyr Cys Leu Gln Tyr Asp Asn Leu Trp Thr
                85                  90                  95

Phe Gly Gln Gly Thr Lys Val Glu Ile Lys Arg Thr Val Ala Ala Pro
            100                 105                 110

Ser Val Phe Ile Phe Pro Pro Ser Asp Glu Gln Leu Lys Ser Gly Thr
        115                 120                 125

Ala Ser Val Val Cys Leu Leu Asn Asn Phe Tyr Pro Arg Glu Ala Lys
    130                 135                 140

Val Gln Trp Lys Val Asp Asn Ala Leu Gln Ser Gly Asn Ser Gln Glu
145                 150                 155                 160

Ser Val Thr Glu Gln Asp Ser Lys Asp Ser Thr Tyr Ser Leu Ser Ser
                165                 170                 175

Thr Leu Thr Leu Ser Lys Ala Asp Tyr Glu Lys His Lys Val Tyr Ala
            180                 185                 190

Cys Glu Val Thr His Gln Gly Leu Ser Ser Pro Val Thr Lys Ser Phe
        195                 200                 205

Asn Arg Gly Glu Cys
    210

<210>  33
<211>  213
<212>  PRT
<213>  Artificial Sequence

<220>
<223>  Light chain NZM var4

<400>  33

Asp Ile Gln Met Thr Gln Ser Pro Ser Ser Leu Ser Ala Ser Val Gly
1               5                   10                  15

Asp Arg Val Thr Ile Thr Cys Lys Thr Ser Gln Asp Ile Asn Lys Tyr
            20                  25                  30

Met Ala Trp Tyr Gln Gln Thr Pro Gly Lys Ala Pro Arg Leu Leu Ile
        35                  40                  45

His Tyr Thr Lys Ser Asn Gln Ser Gly Ile Pro Ser Arg Phe Ser Gly
    50                  55                  60

Ser Gly Ser Gly Arg Asp Tyr Thr Phe Thr Ile Ser Ser Leu Gln Pro
65                  70                  75                  80

Glu Asp Ile Ala Thr Tyr Tyr Cys Leu Gln Tyr Asp Asn Leu Trp Thr
                85                  90                  95

Phe Gly Gln Gly Thr Lys Val Glu Ile Lys Arg Thr Val Ala Ala Pro
            100                 105                 110

Ser Val Phe Ile Phe Pro Pro Ser Asp Glu Gln Leu Lys Ser Gly Thr
        115                 120                 125

Ala Ser Val Val Cys Leu Leu Asn Asn Phe Tyr Pro Arg Glu Ala Lys
    130                 135                 140

Val Gln Trp Lys Val Asp Asn Ala Leu Gln Ser Gly Asn Ser Gln Glu
145                 150                 155                 160

Ser Val Thr Glu Gln Asp Ser Lys Asp Ser Thr Tyr Ser Leu Ser Ser
                165                 170                 175

Thr Leu Thr Leu Ser Lys Ala Asp Tyr Glu Lys His Lys Val Tyr Ala
            180                 185                 190

Cys Glu Val Thr His Gln Gly Leu Ser Ser Pro Val Thr Lys Ser Phe
        195                 200                 205

Asn Arg Gly Glu Cys
    210

<210>  34
<211>  213
<212>  PRT
<213>  Artificial Sequence

<220>
<223>  Light chain NZM var5

<400>  34

Asp Ile Gln Met Thr Gln Ser Pro Ser Ser Leu Ser Ala Ser Val Gly
1               5                   10                  15

Asp Arg Val Thr Ile Thr Cys Lys Thr Ser Gln Asp Ile Asn Lys Tyr
            20                  25                  30

Met Ala Trp Tyr Gln Gln Thr Pro Gly Lys Ala Pro Lys Leu Leu Ile
        35                  40                  45

Tyr Glu Ala Ser Asn Leu Gln Ala Gly Val Pro Ser Arg Phe Ser Gly
    50                  55                  60

Ser Gly Ser Gly Arg Asp Tyr Thr Phe Thr Ile Ser Ser Leu Gln Pro
65                  70                  75                  80

Glu Asp Ile Ala Thr Tyr Tyr Cys Leu Gln Tyr Asp Asn Leu Trp Thr
                85                  90                  95

Phe Gly Gln Gly Thr Lys Val Glu Ile Lys Arg Thr Val Ala Ala Pro
            100                 105                 110

Ser Val Phe Ile Phe Pro Pro Ser Asp Glu Gln Leu Lys Ser Gly Thr
        115                 120                 125

Ala Ser Val Val Cys Leu Leu Asn Asn Phe Tyr Pro Arg Glu Ala Lys
    130                 135                 140

Val Gln Trp Lys Val Asp Asn Ala Leu Gln Ser Gly Asn Ser Gln Glu
145                 150                 155                 160

Ser Val Thr Glu Gln Asp Ser Lys Asp Ser Thr Tyr Ser Leu Ser Ser
                165                 170                 175

Thr Leu Thr Leu Ser Lys Ala Asp Tyr Glu Lys His Lys Val Tyr Ala
            180                 185                 190

Cys Glu Val Thr His Gln Gly Leu Ser Ser Pro Val Thr Lys Ser Phe
        195                 200                 205

Asn Arg Gly Glu Cys
    210

<210>  35
<211>  327
<212>  PRT
<213>  Artificial Sequence

<220>
<223>  heavy chain constant region

<400>  35

Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Lys Thr
65                  70                  75                  80

Tyr Thr Cys Asn Val Asp His Lys Pro Ser Asn Thr Lys Val Asp Lys

85                  90                  95

Arg Val Glu Ser Lys Tyr Gly Pro Pro Cys Pro Ser Cys Pro Ala Pro
            100                 105                 110

Glu Phe Leu Gly Gly Pro Ser Val Phe Leu Phe Pro Pro Lys Pro Lys
        115                 120                 125

Asp Thr Leu Met Ile Ser Arg Thr Pro Glu Val Thr Cys Val Val Val
    130                 135                 140

Asp Val Ser Gln Glu Asp Pro Glu Val Gln Phe Asn Trp Tyr Val Asp
145                 150                 155                 160

Gly Val Glu Val His Asn Ala Lys Thr Lys Pro Arg Glu Glu Gln Phe
                165                 170                 175

Asn Ser Thr Tyr Arg Val Val Ser Val Leu Thr Val Leu His Gln Asp
            180                 185                 190

Trp Leu Asn Gly Lys Glu Tyr Lys Cys Lys Val Ser Asn Lys Gly Leu
        195                 200                 205

Pro Ser Ser Ile Glu Lys Thr Ile Ser Lys Ala Lys Gly Gln Pro Arg
    210                 215                 220

Glu Pro Gln Val Tyr Thr Leu Pro Pro Ser Gln Glu Glu Met Thr Lys
225                 230                 235                 240

Asn Gln Val Ser Leu Thr Cys Leu Val Lys Gly Phe Tyr Pro Ser Asp
                245                 250                 255

Ile Ala Val Glu Trp Glu Ser Asn Gly Gln Pro Glu Asn Asn Tyr Lys
            260                 265                 270

Thr Thr Pro Pro Val Leu Asp Ser Asp Gly Ser Phe Phe Leu Tyr Ser
        275                 280                 285

Arg Leu Thr Val Asp Lys Ser Arg Trp Gln Glu Gly Asn Val Phe Ser
    290                 295                 300

Cys Ser Val Met His Glu Ala Leu His Asn His Tyr Thr Gln Lys Ser
305                 310                 315                 320

Leu Ser Leu Ser Leu Gly Lys
                325

<210>  36
<211>  107
<212>  PRT
<213>  Artificial Sequence

<220>
<223>  Light chain constant region

<400>  36